# EUROPEAN PATENT SPECIFICATION

(11) **EP 2 500 423 B1**
(45) Date of publication and mention of the grant of the patent: **17.06.2015**
(21) Application number: 12156940.4
(22) Date of filing: 26.02.2003
(51) Int. Cl.: C12N 9/28, C12N 9/30, C12N 15/74, C12Q 1/68, G01N 33/53

(54) **Amylases producing an altered immunogenic response and methods of making and using the same**
Amylasen, die eine veränderte immunogene Reaktion bewirken, und Verfahren zu ihrer Herstellung und Verwendung
Amylases produisant une réponse immunogène altérée et leurs procédés de fabrication et d'utilisation

(43) Date of publication of application: 19.09.2012
(62) Divisional of application: 03709323.4
(73) Proprietor: Danisco US Inc., Palo Alto, California 94304 (US)
(72) Inventor: Harding, Fiona, A., Santa Clara, CA 95050 (US)
(74) Representative: Forrest, Graham Robert

(56) References cited:
- EP-A2- 1 277 762
- WO-A-01/59130
- WO-A-94/18314
- WO-A-03/014358
- WO-A2-02/10355
- WO-A2-96/39528
- FR-A1- 2 676 456
- US-A- 5 830 837

## Description

### FIELD OF THE INVENTION

The present invention relates to amylase variants that exhibit reduced immunogenic responses, as compared to the parental proteins. The present invention also relates to DNA molecules that encode novel amylase variants, host cells comprising DNA encoding novel amylase variants, as well as methods for making amylases less allergenic/immunogenic. In addition, the present invention relates to various compositions that comprise these amylase variants that are less immunogenic than the wild-type amylases.

### BACKGROUND OF THE INVENTION

Proteins used in industrial, pharmaceutical and commercial applications are of increasing prevalence and importance. However, this has resulted in the sensitization of numerous individuals to these proteins, resulting in the widespread occurrence of allergic reactions to these proteins. For example, some proteins are associated with hypersensitivity reactions in certain individuals. As a result, despite the usefulness of proteins in industry (*e.g*., in laundry detergents, cosmetics, textile treatment etc.), as well as the extensive research performed in the field to provide improved enzymes (*e.g*., with more effective stain removal under typical laundry conditions), the use of enzymes in industry has been problematic.

Much work has been done to alleviate these problems. Strategies explored to reduce immunogenic potential of enzyme use include improved production processes which reduce potential contact by controlling and minimizing workplace concentrations of dust particles and/or aerosol carrying airborne proteins, improved granulation processes which reduce the amount of dust or aerosol actually produced from the protein product, and improved recovery processes to reduce the level of potentially allergenic/immunogenic contaminants in the final product. However, efforts to reduce the allergenicity/immunogenicity of proteins themselves have been relatively unsuccessful. Alternatively, efforts have been made to mask epitopes in enzymes which are recognized by immunoglobulin E (IgE) in hypersensitive individuals (*See*, PCT Publication No. WO 92/10755; WO 94/10191; WO 96/17929; WO 99/49056; and WO 01/07578), or to enlarge or change the nature of the antigenic determinants by attaching polymers or peptides/proteins to the problematic enzyme.

While some studies have provided methods of reducing the allergenicity/immunogenicity of certain proteins and identification of epitopes which cause allergic reactions in some individuals, the assays used to identify these epitopes generally involve measurement of IgE and IgG in the sera of those who have been previously exposed to the antigen. However, once an Ig reaction has been initiated, sensitization has already occurred. Accordingly, there is a need to identify proteins which produce an enhanced immunologic response, as well as a need to produce proteins which produce a reduced immunologic response.

### SUMMARY OF THE INVENTION

The invention provides a method of reducing immunogenicity of a precursor microbial amylase, comprising modifying at least one amino acid at a position which is equivalent to a position in an epitope defined by SEQ ID NO: 5 of *Bacillus licheniformis* amylase having the amino acid sequence of SEQ ID NO: 1 to produce a variant amylase, whereby said variant amylase is less immunogenic than the precursor amylase.

The precursor microbial amylase may be from a member of the genus *Bacillus,* e.g. from *Bacillus licheniformis.* It may have the sequence of SEQ ID NO: 1.

Said amylase may be obtained from a member of the genus *Bacillus,* e.g. from the group consisting of *B. subtilis, B. licheniformis, B. lentus, B. brevis, B. stearotherophilus, B. alkalophilus, B. amyloliquefaciens, B. clausii, B. halodurans, B. megaterium, B. coagulans, B. circulans, B. lautus and B. thuringiensis.*

The amylase may comprise at least a portion of the sequence set forth in SEQ ID NO: 1.

In some embodiments the amylase is modified by substituting the amino acid sequence of said T-cell epitope with an analogous sequence from a homolog of said amylase, wherein said substitution substantially mimics the major tertiary structure attributes of the T-cell epitope.

### DESCRIPTION OF THE FIGURES

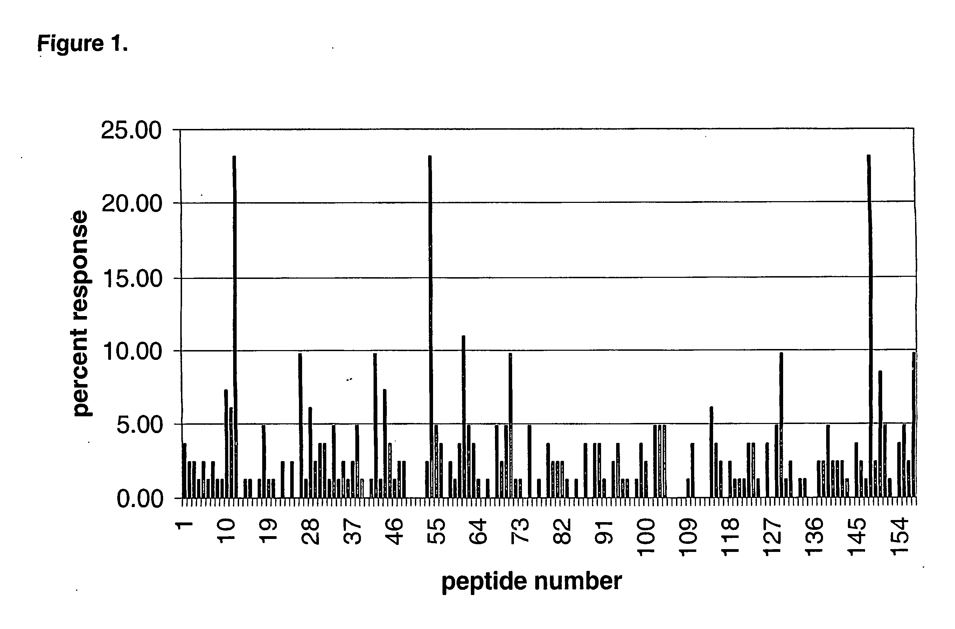

Figure 1 provides a graph showing the per cent responders to the *B*. *licheniformis* amylase peptides.

### DESCRIPTION OF THE INVENTION

The present invention provides methods for reducing immunogenicity of a precursor microbial amylase

### Definitions

Unless defined otherwise herein, all technical and scientific terms used herein have the same meaning as commonly understood by one of ordinary skill in the art to which this invention pertains. For example, Singleton and Sainsbury, Dictionary of Microbiology and Molecular Biology, 2d Ed., John Wiley and Sons, NY (1994); and Hale and Marham, The Harper Collins Dictionary of Biology, Harper Perennial, NY (1991) provide those of skill in the art with a general dictionaries of many of the terms used in the invention. Although any methods and materials similar or equivalent to those described herein find use in the practice of the present invention, the preferred methods and materials are described herein. Accordingly, the terms defined immediately below are more fully described by reference to the Specification as a whole. Also, as used herein, the singular "a", "an" and "the" includes the plural reference unless the context clearly indicates otherwise.

As used herein, "amylase" refers to any enzyme that breaks down (*i.e.*, hydrolyzes) starch or glycogen to dextrins, maltose, and/or glucose. The term encompasses any of the various types of amylases known in the art, including alpha amylase (α-amylase), beta amylase (β-amylase), and glucoamylases. However, in particularly preferred embodiments, the amylase of the present invention comprises α-amylase. The term encompasses naturally-occurring, as well as recombinant amylases.

As used herein, "human amylase" refers to proteins of human origin that exhibit amylase type catalytic activity (*e.g*., the kexin family of human-derived amylases). Additionally, derivatives, variants, and homologs of proteins provided herein, including those from non-human sources such as mouse or rabbit, which retain the essential activity of the enzymes are encompassed by the present invention.

As used herein, "starch" refers to any starch-containing materials. In particular, the term refers to various plant-based materials, including but not limited to wheat, barley, potato, sweet potato, tapioca, corn, maize, cassava, milo, rye, and brans. Indeed, it is not intended that the present invention be limited to any particular type and/or source of starch. In general, the term refers to any material comprised of the complex polysaccharide carbohydrates of plants, comprised of amylose and amylopectin, with the formula (C₆H₁₀O₅)*ₓ,* wherein "*x*" can be any number.

As used herein, the term "enzymatic conversion" refers to the modification of a carbon substrate to an intermediate or the modification of an intermediate to an end-product by contacting the substrate or intermediate with an enzyme. Contact may be made by directly exposing the substrate or intermediate to the appropriate enzyme. Alternatively, contacting comprises exposing the substrate or intermediate to an organism that expresses and/or excretes the enzyme, and/or metabolizes the desired substrate and/or intermediate to the desired intermediate and/or end-product, respectively.

As used herein, "monosaccharide" refers to any compound having an empirical formula of (CH₂O)_{n,} wherein n is 3-7, and preferably 5-7. The term may refer to "simple sugars" that consist of a single polyhydroxy aldehyde or ketone unit. The term encompasses, but is not limited to such compounds as glucose, galactose, and fructose.

As used herein, "disaccharide" refers to any compound that comprises two covalently linked monosaccharide units. The term encompasses, but is not limited to such compounds as sucrose, lactose and maltose.

As used herein, "oligosaccharide" refers to any compound having 2 -10 The monosaccharide units joined in glycosidic linkages. The term preferably refers to short chains of monosaccharide units joined together by covalent bonds.

As used herein, "polysaccharide" refers to any compound having multiple monosaccharide units joined in a linear or branched chain. Preferably, the term refers to long chains with hundreds or thousands of monosaccharide units. Some polysaccharides, such as cellulose have linear chains, while others (*e.g*., glycogen) have branched chains. Among the most abundant polysaccharides are starch and cellulose, which consist of recurring glucose units (although these compounds differ in how the glucose units are linked).

As used herein, "the genus *Bacillus*" includes all members known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus,* and *B*. *thuringiensis.* It is recognized that the genus *Bacillus* continues to undergo taxonomical reorganization. Thus, it is intended that the genus include species that have been reclassified, including but not limited to such organisms as *B*. *stearothermophilus,* which is now named "*Geobacillus stearothermophilus*." The production of resistant endospores in the presence of oxygen is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus*, *Thermobacillus*, *Ureibacillus,* and *Virgibacillus.*

As used herein, "wild-type" and "native" proteins are those found in nature. The terms "wild-type sequence," and "wild-type gene" are used interchangeably herein, to refer to a sequence that is native or naturally occurring in a host cell. The wild-type sequence may refer to a sequence of interest that is the starting point of a protein engineering project. The genes encoding the naturally-occurring (*i.e.*, precursor) protein may be obtained in accord with the general methods known to those skilled in the art. The methods generally comprise synthesizing labeled probes having putative sequences encoding regions of the protein of interest, preparing genomic libraries from organisms expressing the protein, and screening the libraries for the gene of interest by hybridization to the probes. Positively hybridizing clones are then mapped and sequenced.

"Recombinant amylase" refers to an amylase in which the DNA sequence encoding amylase is modified to produce a variant (or mutant) DNA sequence which encodes the substitution, deletion or insertion of one or more amino acids in the naturally-occurring amino acid sequence. Suitable methods to produce such modification, and which may be combined with those disclosed herein, include those disclosed in US Patent 4,760,025 (US RE 34,606), US Patent 5,204,015 and US Patent 5,185,258.

The term "sample" as used herein is used in its broadest sense. However, the term is preferably used in reference to a sample (*e.g*., an aliquot) that comprises a peptide (*i.e.*, a peptide within a pepset, that comprises a sequence of a protein of interest) that is being analyzed, identified, modified, and/or compared with other peptides. Thus, in most cases, this term is used in reference to material that includes a protein or peptide that is of interest.

As used herein, "Stimulation Index" (SI) refers to a measure of the T-cell proliferative response of a peptide compared to a control. The SI is calculated by dividing the average CPM (counts per minute) obtained in testing the CD4⁺ T-cell and dendritic cell culture containing a peptide by the average CPM of the control culture containing dendritic cells and CD4⁺ T-cells but without the peptides. This value is calculated for each donor and for each peptide. While SI values of between about 1.5 to 4.5 may be used to indicate a positive response, the preferred SI value to indicate a positive response is between 2.5 and 3.5, inclusive, preferably between 2.7 and 3.2 inclusive, and more preferably between 2.9 and 3.1 inclusive. The most preferred embodiments described herein use a SI value of 2.95.

As used herein, the term "dataset" as used herein refers to compiled data for a set of peptides and a set of donors for each protein.

As used herein, the term "pepset" refers to the set of peptides obtained from each test protein (*i.e.*, protein of interest). These peptides in the pepset (or "peptide sets") are tested with cells from each donor.

As used herein, the terms "purified" and "isolated" refer to the removal of contaminants from a sample. For example, amylases are purified by removal of contaminating proteins and other compounds within a solution or preparation that are not amylases. Recombinant amylases may be expressed in bacterial host cells and these recombinant amylases are purified by the removal of other host cell constituents; the percent of recombinant amylase polypeptides is thereby increased in the sample.

As used herein, "background level" and "background response" refer to the average percent of responders to any given peptide in the dataset for any tested protein. This value is determined by averaging the percent responders for all peptides in the set, as compiled for all the tested donors. As an example, a 3% background response would indicate that on average there would be three positive (SI greater than 2.95) responses for any peptide in a dataset when tested on 100 donors.

As used herein, "antigen presenting cell" ("APC") refers to a cell of the immune system that presents antigen on its surface, such that the antigen is recognizable by receptors on the surface of T-cells. Antigen presenting cells include, but are not limited to dendritic cells, interdigitating cells, activated B-cells and macrophages.

The term "lymphoid" when used in reference to a cell line or a cell, means that the cell line or cell is derived from the lymphoid lineage and includes cells of both the B and the T lymphocyte lineages.

As used herein, the terms "T lymphocyte" and "T-cell," encompass any cell within the T lymphocyte lineage from T-cell precursors (including Thy1 positive cells which have not rearranged the T cell receptor genes) to mature T cells (*i.e.*, single positive for either CD4 or CD8, surface TCR positive cells).

As used herein, the terms "B lymphocyte" and "B-cell" encompasses any cell within the B-cell lineage from B-cell precursors, such as pre-B-cells (B220⁺ cells which have begun to rearrange Ig heavy chain genes), to mature B-cells and plasma cells.

As used herein, "CD4⁺ T-cell" and "CD4 T-cell" refer to helper T-cells, while "CD8⁺ T-cell" and CD8 T-cell" refer to cytotoxic T-cells.

As used herein, "B-cell proliferation," refers to the number of B-cells produced during the incubation of B-cells with the antigen presenting cells, with or without antigen.

As used herein, "baseline B-cell proliferation," as used herein, refers to the degree of B-cell proliferation that is normally seen in an individual in response to exposure to antigen presenting cells in the absence of peptide or protein antigen. For the purposes herein, the baseline B-cell proliferation level is determined on a per sample basis for each individual as the proliferation of B-cells in the absence of antigen.

As used herein, "B-cell epitope," refers to a feature of a peptide or protein that is recognized by a B-cell receptor in the immunogenic response to the peptide comprising that antigen (*i.e.*, the immunogen).

As used herein, "altered B-cell epitope," refers to an epitope amino acid sequence which differs from the precursor peptide or peptide of interest, such that the variant peptide of interest produces different (*i.e.*, altered) immunogenic responses in a human or another animal. It is contemplated that an altered immunogenic response includes altered immunogenicity and/or allergenicity (*i.e.*, an either increased or decreased overall immunogenic response). The altered B-cell epitope may comprise substitution and/or deletion of an amino acid selected from those residues within the identified epitope. Alternatively, the altered B-cell epitope comprises an addition of one or more residues within the epitope.

As used herein "T-cell epitope" means a feature of a peptide or protein that is recognized by a T-cell receptor in the initiation of an immunologic response to the peptide comprising that antigen. Recognition of a T-cell epitope by a T-cell is generally believed to be via a mechanism wherein T-cells recognize peptide fragments of antigens which are bound to class I or class II Major Histocompatibility Complex (MHC) molecules expressed on antigen-presenting cells (See *e.g.*, Moeller (ed.), Immunol. Rev., 98:187 [1987]). The epitopes or epitopic fragments identified as described herein may find use in the detection of antigen presenting cells having MHC molecules capable of binding and displaying the epitopes or fragments. The epitopes/epitopic fragments may further comprise a detectable label (*i.e.*, a marker) that facilitates the identification of cells that bind and/or display the epitope/epitopic fragment of interest.

As used herein, "T-cell proliferation," refers to the number of T-cells produced during the incubation of T-cells with the antigen presenting cells, with or without antigen.

"Baseline T-cell proliferation," as used herein, refers to the degree of T-cell proliferation that is normally seen in an individual in response to exposure to antigen presenting cells in the absence of peptide or protein antigen. For the purposes herein, the baseline T-cell proliferation level is determined on a per sample basis for each individual as the proliferation of T-cells in response to antigen presenting cells in the absence of antigen.

As used herein "altered immunogenic response," refers to an increased or reduced immunogenic response. Proteins and peptides exhibit an "increased immunogenic response" when the T-cell and/or B-cell response they evoke is greater than that evoked by a parental (*e.g*., precursor) protein or peptide (*e.g*., the protein of interest). The net result of this higher response is an increased antibody response directed against the variant protein or peptide. Proteins and peptides exhibit a "reduced immunogenic response" when the T-cell and/or B-cell response they evoke is less than that evoked by a parental (*e.g*., precursor) protein or peptide. In preferred embodiments, the net result of this lower response is a reduced antibody response directed against the variant protein or peptide. In some preferred embodiments, the parental protein is a wild-type protein or peptide.

As used herein, an "*in vivo* reduction in immunogenicity" refers to an exhibited decrease in the immunogenic response as determined by an assay that occurs at least in part, within a living organism, (*e.g*., requires the use of an living animal). Exemplary "*in vivo"* assays include determination of altered immunogenic responses in mouse models.

As used herein, an "*in vitro* reduction in immunogenicity" refers an exhibited decrease in the immunogenic response as determined by an assay that occurs in an artificial environment outside of a living organism (*i.e.*, does not require use of a living animal). Exemplary *in vitro* assays include testing the proliferative responses by human peripheral blood mononuclear cells to a peptide of interest.

As used herein, the term "significant epitope" refers to an epitope (*i.e.*, a T-cell and/or B-cell epitope) wherein the response rate within the tested donor pool is equal to or greater than about three times the background response rate.

As used herein, a "weakly significant epitope" refers to an epitope (*i.e.*, a T-cell and/or B-cell epitope), wherein the response rate within the tested donor pool is greater than the background response rate, but less than about three times the background rate.

As used herein, "protein of interest," refers to a protein which is being analyzed, identified and/or modified. Naturally-occurring, as well as recombinant proteins find use in the present invention.

As used herein, "protein" refers to any composition comprised of amino acids and recognized as a protein by those of skill in the art. The terms "protein," "peptide" and polypeptide are used interchangeably herein. Wherein a peptide is a portion of a protein, those skill in the art understand the use of the term in context. The term "protein" encompasses mature forms of proteins, as well as the pro- and prepro-forms of related proteins. Prepro forms of proteins comprise the mature form of the protein having a prosequence operably linked to the amino terminus of the protein, and a "pre-" or "signal" sequence operably linked to the amino terminus of the prosequence.

The variants described herein include the mature forms of protein variants, as well as the pro- and prepro- forms of such protein variants. The prepro- forms are the preferred construction since this facilitates the expression, secretion and maturation of the protein variants.

As used herein, "prosequence" refers to a sequence of amino acids bound to the N-terminal portion of the mature form of a protein which when removed results in the appearance of the "mature" form of the protein. Many enzymes are found in nature as translational proenzyme products and, in the absence of post-translational processing, are expressed in this fashion.

As used herein, "signal sequence" and "presequence" refer to any sequence of amino acids bound to the N-terminal portion of a protein or to the N-terminal portion of a pro-protein which may participate in the secretion of the mature or pro forms of the protein. This definition of signal sequence is a functional one and is intended to include all those amino acid sequences encoded by the N-terminal portion of the protein gene that participate in the effectuation of the secretion of protein under native conditions. Such sequences may be utilized to effect the secretion of the protein variants described herein.

As used herein, a "prepro" form of a protein variant consists of the mature form of the protein having a prosequence operably linked to the amino terminus of the protein and a "pre" or "signal" sequence operably linked to the amino terminus of the prosequence.

As used herein, functionally similar proteins are considered to be "related proteins." These proteins may be derived from a different genus and/or species, including differences between classes of organisms (*e.g*., a bacterial protein and a fungal protein). These proteins may be derived from a different genus and/or species (*e.g., B. subtilis* amylase and *B. licheniformis* amylase), including differences between classes of organisms (*e.g*., a bacterial amylase and a fungal amylase). Related proteins may be provided from the same species.

As used herein, the term "derivative" refers to a protein which is derived from a precursor protein by addition of one or more amino acids to either or both the C- and N-terminal end(s), substitution of one or more amino acids at one or a number of different sites in the amino acid sequence, and/or deletion of one or more amino acids at either or both ends of the protein or at one or more sites in the amino acid sequence, and/or insertion of one or more amino acids at one or more sites in the amino acid sequence. The preparation of a protein derivative is preferably achieved by modifying a DNA sequence which encodes for the native protein, transformation of that DNA sequence into a suitable host, and expression of the modified DNA sequence to form the derivative protein.

Related (and derivative) proteins comprise "variant proteins." In preferred embodiments, variant proteins differ from a parent protein and one another by a small number of amino acid residues. The number of differing amino acid residues may be one or more, preferably 1, 2, 3, 4, 5, 10, 15, 20, 30, 40, 50, or more amino acid residues. In one preferred embodiment, the number of different amino acids between variants is between 1 and 10. In particularly preferred embodiments, related proteins and particularly variant proteins comprise at least 50%, 60%, 65%. 70%, 75%, 80%, 85%, 90%, 95%, 97%, 98%, or 99% amino acid sequence identity. Additionally, a related protein or a variant protein as used herein, refers to a protein that differs from another related protein or a parent protein in the number of prominent regions. For example, variant proteins may have 1, 2, 3, 4, 5, or 10 corresponding prominent regions that differ from the parent protein.

In one embodiment, the prominent corresponding region of a variant produces only a background level of immunogenic response. Some of the residues identified for substitution, insertion or deletion are conserved residues whereas others are not. In the case of residues which are not conserved, the replacement of one or more amino acids is limited to substitutions which produce a variant which has an amino acid sequence that does not correspond to one found in nature. In the case of conserved residues, such replacements should not result in a naturally-occurring sequence.

In some embodiments, modification is preferably made to the "precursor DNA sequence" which encodes the amino acid sequence of the precursor enzyme, but can be by the manipulation of the precursor protein. In the case of residues which are not conserved, the replacement of one or more amino acids is limited to substitutions which produce a variant which has an amino acid sequence that does not correspond to one found in nature. In the case of conserved residues, such replacements should not result in a naturally-occurring sequence. Derivatives provided by the present invention further include chemical modification(s) that change the characteristics of the amylase.

Characteristics of variant amylases may be determined by methods known to those skilled in the art. Exemplary characteristics include, but are not limited to thermal stability, alkaline stability, and stability of the particular amylase, various substrate, buffer solutions and/or product formulations. In combination with enzyme stability assays, variant amylases obtained through random mutagenesis can be identified which demonstrate either increased or decreased alkaline or thermal stability while maintaining enzymatic activity.

Alkaline stability can be measured either by known procedures or by the methods described herein. A substantial change in alkaline stability is evidenced by at least about a 5% or greater increase or decrease (it is preferably an increase) in the half-life of the enzymatic activity of a mutant when compared to the precursor protein.

Thermal stability can be measured either by known procedures or by the methods described herein. A substantial change in thermal stability is evidenced by at least about a 5% or greater increase or decrease (it is preferably an increase) in the half-life of the catalytic activity of a mutant when exposed to a relatively high temperature and neutral pH as compared to the precursor protein.

In some preferred embodiments, the amylase gene is ligated into an appropriate expression plasmid. The cloned amylase gene is then used to transform or transfect a host cell in order to express the amylase gene. This plasmid may replicate in hosts in the sense that it contains the well-known elements necessary for plasmid replication or the plasmid may be designed to integrate into the host chromosome. The necessary elements are provided for efficient gene expression (*e.g*., a promoter operably linked to the gene of interest). In some embodiments, these necessary elements are supplied as the gene's own homologous promoter if it is recognized, (*i.e.*, transcribed, by the host), a transcription terminator (a polyadenylation region for eukaryotic host cells) which is exogenous or is supplied by the endogenous terminator region of the amylase gene. In some embodiments, a selection gene such as an antibiotic resistance gene that enables continuous cultural maintenance of plasmid-infected host cells by growth in antimicrobial-containing media is also included.

The following cassette mutagenesis method may be used to facilitate the construction of the amylase variants of the present invention, although other methods may be used. First, the naturally-occurring gene encoding the amylase is obtained and sequenced in whole or in part. Then, the sequence is scanned for a point at which it is desired to make a mutation (deletion, insertion or substitution) of one or more amino acids in the encoded amylase. The sequences flanking this point are evaluated for the presence of restriction sites for replacing a short segment of the gene with an oligonucleotide pool which when expressed will encode various mutants. Such restriction sites are preferably unique sites within the protein gene so as to facilitate the replacement of the gene segment. However, any convenient restriction site which is not overly redundant in the amylase gene may be used, provided the gene fragments generated by restriction digestion can be reassembled in proper sequence. If restriction sites are not present at locations within a convenient distance from the selected point (from 10 to 15 nucleotides), such sites are generated by substituting nucleotides in the gene in such a fashion that neither the reading frame nor the amino acids encoded are changed in the final construction. Mutation of the gene in order to change its sequence to conform to the desired sequence is accomplished by M13 primer extension in accord with generally known methods. The task of locating suitable flanking regions and evaluating the needed changes to arrive at two convenient restriction site sequences is made routine by the redundancy of the genetic code, a restriction enzyme map of the gene and the large number of different restriction enzymes. Note that if a convenient flanking restriction site is available, the above method need be used only in connection with the flanking region which does not contain a site.

Once the naturally-occurring DNA or synthetic DNA is cloned, the restriction sites flanking the positions to be mutated are digested with the cognate restriction enzymes and a plurality of end termini-complementary oligonucleotide cassettes are ligated into the gene. The mutagenesis is simplified by this method because all of the oligonucleotides can be synthesized so as to have the same restriction sites, and no synthetic linkers are necessary to create the restriction sites.

As used herein, "corresponding to," refers to a residue at the enumerated position in a protein or peptide, or a residue that is analogous, homologous, or equivalent to an enumerated residue in a protein or peptide.

As used herein, "corresponding region," generally refers to an analogous position along related proteins or a parent protein.

The terms "nucleic acid molecule encoding," "DNA sequence encoding," and "DNA encoding" refer to the order or sequence of deoxyribonucleotides along a strand of deoxyribonucleic acid. The order of these deoxyribonucleotides determines the order of amino acids along the polypeptide (protein) chain. The DNA sequence thus codes for the amino acid sequence.

As used herein, the term "analogous sequence" refers to a sequence within a protein that provides similar function, tertiary structure, and/or conserved residues as the protein of interest (*i.e.*, typically the original protein of interest). In particularly preferred embodiments, the analogous sequence involves sequence(s) at or near an epitope. For example, in epitope regions that contain an alpha helix or a beta sheet structure, the replacement amino acids in the analogous sequence preferably maintain the same specific structure. The term also refers to nucleotide sequences, as well as amino acid sequences. In some embodiments, analogous sequences are developed such that the replacement amino acids show a similar function, the tertiary structure and/or conserved residues to the amino acids in the protein of interest at or near the epitope. Thus, where the epitope region contains, for example, an alpha-helix or a beta-sheet structure, the replacement amino acids preferably maintain that specific structure.

As used herein, "homologous protein" refers to a protein (*e.g*., amylase) that has similar action, structure, antigenic, and/or immunogenic response as a protein of interest (*e.g*., an amylase from another source). It is not intended that homologs be necessarily related evolutionarily. Thus, it is intended that the term encompass the same functional protein obtained from different species. In some preferred embodiments, it is desirable to identify a homolog that has a tertiary and/or primary structure similar to the protein of interest, as replacement for the epitope in the protein of interest with an analogous segment from the homolog will reduce the disruptiveness of the change. Thus, in most cases, closely homologous proteins provide the most desirable sources of epitope substitutions. Alternatively, it is advantageous to look to human analogs for a given protein. For example, in some embodiments, substituting a specific epitope in one amylase with a sequence from another amylase or other species' amylase results in the production of amylase with reduced immunogenicity. In some preferred embodiments, the amylase homologs of the present invention have tertiary and/or primary structures substantially similar to wild-type amylase. A significant amylase epitope may be replaced with an analogous segment from a homologous enzyme. This type of replacement may reduce the disruptiveness of the change in the parent amylase. In most cases, closely homologous proteins provide the most desirable source of epitope substitutions.

As used herein, "homologous genes" refers to at least a pair of genes from different, but usually related species, which correspond to each other and which are identical or very similar to each other. The term encompasses genes that are separated by speciation (*i.e.*, the development of new species) (*e.g*., orthologous genes), as well as genes that have been separated by genetic duplication (*e.g*., paralogous genes). These genes encode "homologous proteins."

As used herein, "ortholog" and "orthologous genes" refer to genes in different species that have evolved from a common ancestral gene (*i.e.*, a homologous gene) by speciation. Typically, orthologs retain the same function in during the course of evolution. Identification of orthologs finds use in the reliable prediction of gene function in newly sequenced genomes.

As used herein, "paralog" and "paralogous genes" refer to genes that are related by duplication within a genome. While orthologs retain the same function through the course of evolution, paralogs evolve new functions, even though some functions are often related to the original one. Examples of paralogous genes include, but are not limited to genes encoding trypsin, chymotrypsin, elastase, and thrombin, which are all serine proteinases and occur together within the same species.

As used herein, "wild-type" and "native" proteins are those found in nature. The terms "wild-type sequence," and "wild-type gene" are used interchangeably herein, to refer to a sequence that is native or naturally occurring in a host cell. In some embodiments, the wild-type sequence refers to a sequence of interest that is the starting point of a protein engineering project. The genes encoding the naturally-occurring (*i.e.*, precursor) protein may be obtained in accord with the general methods known to those skilled in the art. The methods generally comprise synthesizing labeled probes having putative sequences encoding regions of the protein of interest, preparing genomic libraries from organisms expressing the protein, and screening the libraries for the gene of interest by hybridization to the probes. Positively hybridizing clones are then mapped and sequenced.

The term "recombinant DNA molecule" as used herein refers to a DNA molecule that is comprised of segments of DNA joined together by means of molecular biological techniques.

The term "recombinant oligonucleotide" refers to an oligonucleotide created using molecular biological manipulations, including but not limited to, the ligation of two or more oligonucleotide sequences generated by restriction enzyme digestion of a polynucleotide sequence, the synthesis of oligonucleotides (*e.g*., the synthesis of primers or oligonucleotides) and the like.

The degree of homology between sequences may be determined using any suitable method known in the art (*See e.g*., Smith and Waterman, Adv. Appl. Math., 2:482 [1981]; Needleman and Wunsch, J. Mol. Biol., 48:443 [1970]; Pearson and Lipman, Proc. Natl. Acad. Sci. USA 85:2444 [1988]; programs such as GAP, BESTFIT, FASTA, and TFASTA in the Wisconsin Genetics Software Package (Genetics Computer Group, Madison, WI); and Devereux et al., Nucl. Acid Res., 12:387-395 [1984]).

For example, PILEUP is a useful program to determine sequence homology levels. PILEUP creates a multiple sequence alignment from a group of related sequences using progressive, pairwise alignments. It can also plot a tree showing the clustering relationships used to create the alignment. PILEUP uses a simplification of the progressive alignment method of Feng and Doolittle, (Feng and Doolittle, J. Mol. Evol., 35:351-360 [1987]). The method is similar to that described by Higgins and Sharp (Higgins and Sharp, CABIOS 5:151-153 [1989]). Useful PILEUP parameters including a default gap weight of 3.00, a default gap length weight of 0.10, and weighted end gaps. Another example of a useful algorithm is the BLAST algorithm, described by Altschul *et al.,* (Altschul et al., J. Mol. Biol., 215:403-410, [1990]; and Karlin et al., Proc. Natl. Acad. Sci. USA 90:5873-5787 [1993]). One particularly useful BLAST program is the WU-BLAST-2 program (*See*, Altschul et al., Meth. Enzymol." 266:460-480 [1996]). parameters "W," "T," and "X" determine the sensitivity and speed of the alignment. The BLAST program uses as defaults a wordlength (W) of 11, the BLOSUM62 scoring matrix (*See*, Henikoff and Henikoff, Proc. Natl. Acad. Sci. USA 89:10915 [1989]) alignments (B) of 50, expectation (E) of 10, M'5, N'-4, and a comparison of both strands.

As used herein, "percent (%) nucleic acid sequence identity" is defined as the percentage of nucleotide residues in a candidate sequence that are identical with the nucleotide residues of the sequence.

As used herein, the term "hybridization" refers to the process by which a strand of nucleic acid joins with a complementary strand through base pairing, as known in the art.

As used herein, "hybridization conditions" refers to the conditions under which hybridization reactions are conducted. These conditions are typically classified by degree of "stringency" of the conditions under which hybridization is measured. The degree of stringency can be based, for example, on the melting temperature (Tm) of the nucleic acid binding complex or probe. For example, "maximum stringency" typically occurs at about Tm-5°C (5° below the Tm of the probe); "high stringency" at about 5-10° below the Tm; "intermediate stringency" at about 10-20° below the Tm of the probe; and "low stringency" at about 20-25° below the Tm. Alternatively, or in addition, hybridization conditions can be based upon the salt or ionic strength conditions of hybridization and/or one or more stringency washes. For example, 6xSSC = very low stringency; 3xSSC = low to medium stringency; 1xSSC = medium stringency; and 0.5xSSC = high stringency. Functionally, maximum stringency conditions may be used to identify nucleic acid sequences having strict identity or near-strict identity with the hybridization probe; while high stringency conditions are used to identify nucleic acid sequences having about 80% or more sequence identity with the probe.

For applications requiring high selectivity, one will typically desire to employ relatively stringent conditions to form the hybrids (*e.g*., relatively low salt and/or high temperature conditions are used).

The phrases "substantially similar and "substantially identical" in the context of at least two nucleic acids or polypeptides typically means that a polynucleotide or polypeptide comprises a sequence that has at least 60% identity, preferably at least 75% sequence identity, more preferably at least 80%, yet more preferably at least 90%, still more preferably 95%, most preferably 97%, sometimes as much as 98% and 99% sequence identity, compared to the reference (*i.e.*, wild-type) sequence. Sequence identity may be determined using known programs such as BLAST, ALIGN, and CLUSTAL using standard parameters. (*See e.g.,* Altschul, et al., J. Mol. Biol. 215:403-410 [1990]; Henikoff et al., Proc. Natl. Acad Sci. USA 89:10915 [1989]; Karin et al., Proc. Natl Acad. Sci USA 90:5873 [1993]; and Higgins et al., Gene 73:237 - 244 [1988]). Software for performing BLAST analyses is publicly available through the National Center for Biotechnology Information. Also, databases may be searched using FASTA (Pearson et al., Proc. Natl. Acad. Sci. USA 85:2444-2448 [1988]). One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative substitution. Another indication that two nucleic acid sequences are substantially identical is that the two molecules hybridize to each other under stringent conditions (*e.g*., within a range of medium to high stringency).

As used herein, "equivalent residues" refers to proteins that share particular amino acid residues. For example, equivalent resides may be identified by determining homology at the level of tertiary structure for a protein (*e.g.*, IFN-β) whose tertiary structure has been determined by x-ray crystallography. Equivalent residues are defined as those for which the atomic coordinates of two or more of the main chain atoms of a particular amino acid residue of the protein having putative equivalent residues and the protein of interest (N on N, CA on CA, C on C and O on O) are within 0.13 nm and preferably 0.1 nm after alignment. Alignment is achieved after the best model has been oriented and positioned to give the maximum overlap of atomic coordinates of non-hydrogen protein atoms of the proteins analyzed. The preferred model is the crystallographic model giving the lowest R factor for experimental diffraction data at the highest resolution available, determined using methods known to those skilled in the art of crystallography and protein characterization/analysis.

Described herein are amylases having altered immunogenicity that are equivalent to those that are derived from the particular microbial strain mentioned. Being "equivalent," in this context, means that the amylases are encoded by a polynucleotide capable of hybridizing to the polynucleotide encoding the amino acid sequence of SEQ ID NO:1 under conditions of medium to high stringency and still retaining the altered immunogenic response to human T-cells. Thus, equivalent amylases may comprise at least 55%, at least 65%, at least 70%, at least 75%, at least 80%, at least 85%, at least 90%, at least 95%, at least 97% or at least 99% identity to the epitope sequences and the variant amylases having such epitopes.

As used herein, the terms "hybrid amylases" and "fusion amylases " refer to proteins that are engineered from at least two different or "parental" proteins. These parental proteins are preferably homologs of one another. For example, a preferred hybrid amylase or fusion protein contains the N-terminus of a protein and the C-terminus of a homolog of the protein. The two terminal ends may preferably be combined to correspond to the full-length active protein. Alternatively, the homologs may share substantial similarity but do not have identical T-cell epitopes. Therefore, an amylase of interest is provided having one or more T-cell epitopes in the C-terminus, but in which the C-terminus is replaced with the C-terminus of a homolog having a less potent T-cell epitope, or fewer or no T-cell epitopes in the C-terminus. Thus, the skilled artisan understands that by being able to identify T-cell epitopes among homologs, a variety of variants producing different immunogenic responses can be formed. Moreover, it is understood that internal portions, and more than one homolog can be used to produce the variants.

The term "regulatory element" as used herein refers to a genetic element that controls some aspect of the expression of nucleic acid sequences. For example, a promoter is a regulatory element which facilitates the initiation of transcription of an operably linked coding region. Additional regulatory elements include splicing signals, polyadenylation signals and termination signals.

As used herein, "expression vector" refers to a DNA construct containing a DNA sequence that is operably linked to a suitable control sequence capable of effecting the expression of the DNA in a suitable host. Such control sequences include a promoter to effect transcription, an optional operator sequence to control such transcription, a sequence encoding suitable mRNA ribosome binding sites and sequences which control termination of transcription and translation. The vector may be a plasmid, a phage particle, or simply a potential genomic insert. Once transformed into a suitable host, the vector may replicate and function independently of the host genome, or may, in some instances, integrate into the genome itself. In the present specification, "plasmid" and "vector" are sometimes used interchangeably as the plasmid is the most commonly used form of vector at present. However, other forms of expression vectors that serve equivalent functions and which are, or become, known in the, art can also be used.

As used herein, "host cells" are generally prokaryotic or eukaryotic hosts which preferably have been manipulated by the methods known in the art (*See e.g.,* U.S. Patent 4,760,025 (RE 34,606)) to render them incapable of secreting enzymatically active endoprotease. A preferred host cell for expressing protein is the *Bacillus* strain BG2036 which is deficient in enzymatically active neutral protein and alkaline protease (subtilisin). The construction of strain BG2036 is described in detail in US Patent 5,264,366. Other host cells for expressing protein include *Bacillus subtilis* I168 (also described in US Patent 4,760,025 (RE 34,606) and US Patent 5,264,366) as well as any suitable *Bacillus* strain, including those within the species of *B*. *licheniformis, B. lentus,* and other *Bacillus* species, etc. It is preferred that, the host cell has been modified such that endogenous amylase is not produced by the host cell.

Host cells are transformed or transfected with vectors constructed using recombinant DNA techniques known in the art. Transformed host cells are capable of either replicating vectors encoding the protein variants or expressing the desired protein variant. In the case of vectors which encode the pre- or prepro-form of the protein variant, such variants, when expressed, are typically secreted from the host cell into the host cell medium.

The term "introduced" in the context of inserting a nucleic acid sequence into a cell, means transformation, transduction or transfection. Means of transformation include protoplast transformation, calcium chloride precipitation, electroporation, naked DNA and the like as known in the art. (See, Chang and Cohen (1979) Mol. Gen. Genet. 168:111 - 115; Smith et al., (1986) Appl. and Env. Microbiol. 51:634; and the review article by Ferrari et al., (1989) Genetics, pages 57 - 72 in Bacillus ed. C. Harwood, Plenum Publishing Corporation).

The term "promoter/enhancer" denotes a segment of DNA which contains sequences capable of providing both promoter and enhancer functions (for example, the long terminal repeats of retroviruses contain both promoter and enhancer functions). The enhancer/promoter may be "endogenous" or "exogenous" or "heterologous." An endogenous enhancer/promoter is one which is naturally linked with a given gene in the genome. An exogenous (heterologous) enhancer/promoter is one which is placed in juxtaposition to a gene by means of genetic manipulation (*i.e.*, molecular biological techniques).

The presence of "splicing signals" on an expression vector often results in higher levels of expression of the recombinant transcript. Splicing signals mediate the removal of introns from the primary RNA transcript and consist of a splice donor and acceptor site (Sambrook et al., Molecular Cloning: A Laboratory Manual, 2nd ed., Cold Spring Harbor Laboratory Press, New York [1989], pp. 16.7-16.8). A commonly used splice donor and acceptor site is the splice junction from the 16S RNA of SV40.

The term "stable transfection" or "stably transfected" refers to the introduction and integration of foreign DNA into the genome of the transfected cell. The term "stable transfectant" refers to a cell which has stably integrated foreign or exogenous DNA into the genomic DNA of the transfected cell.

The terms "selectable marker" or "selectable gene product" as used herein refer to the use of a gene which encodes an enzymatic activity that confers resistance to an antibiotic or drug upon the cell in which the selectable marker is expressed.

As used herein, the terms "amplification" and "gene amplification" refer to a process by which specific DNA sequences are disproportionately replicated such that the amplified gene becomes present in a higher copy number than was initially present in the genome. Selection of cells by growth in the presence of a drug (*e.g*., an inhibitor of an inhibitable enzyme) may result in the amplification of either the endogenous gene encoding the gene product required for growth in the presence of the drug or by amplification of exogenous (*i.e.*, input) sequences encoding this gene product, or both. Gene amplification occurs naturally during development in particular genes such as the amplification of ribosomal genes in amphibian oocytes. Gene amplification may be induced by treating cultured cells with drugs. An example of drug-induced amplification is the methotrexate-induced amplification of the endogenous *dhfr* gene in mammalian cells (Schmike et al., Science 202:1051 [1978]). Selection of cells by growth in the presence of a drug (*e.g*., an inhibitor of an inhibitable enzyme) may result in the amplification of either the endogenous gene encoding the gene product required for growth in the presence of the drug or by amplification of exogenous (*i.e.*, input) sequences encoding this gene product, or both.

"Amplification" is a special case of nucleic acid replication involving template specificity. It is to be contrasted with non-specific template replication (*i.e.*, replication that is template-dependent but not dependent on a specific template). Template specificity is here distinguished from fidelity of replication (*i.e*., synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

As used herein, the term "co-amplification" refers to the introduction into a single cell of an amplifiable marker in conjunction with other gene sequences (*i.e.*, comprising one or more non-selectable genes such as those contained within an expression vector) and the application of appropriate selective pressure such that the cell amplifies both the amplifiable marker and the other, non-selectable gene sequences. The amplifiable marker may be physically linked to the other gene sequences or alternatively two separate pieces of DNA, one containing the amplifiable marker and the other containing the non-selectable marker, may be introduced into the same cell.

As used herein, the terms "amplifiable marker," "amplifiable gene," and "amplification vector" refer to a marker, gene or a vector encoding a gene which permits the amplification of that gene under appropriate growth conditions.

As used herein, the term "amplifiable nucleic acid" refers to nucleic acids which may be amplified by any amplification method. It is contemplated that "amplifiable nucleic acid" will usually comprise "sample template."

As used herein, the term "sample template" refers to nucleic acid originating from a sample which is analyzed for the presence of "target" (defined below). In contrast, "background template" is used in reference to nucleic acid other than sample template which may or may not be present in a sample. Background template is most often inadvertent. It may be the result of carryover, or it may be due to the presence of nucleic acid contaminants sought to be purified away from the sample. For example, nucleic acids from organisms other than those to be detected may be present as background in a test sample.

"Template specificity" is achieved in most amplification techniques by the choice of enzyme. Amplification enzymes are enzymes that, under conditions they are used, will process only specific sequences of nucleic acid in a heterogeneous mixture of nucleic acid. For example, in the case of Qβ replicase, MDV-1 RNA is the specific template for the replicase (*See e.g*., Kacian et al., Proc. Natl. Acad. Sci. USA 69:3038 [1972]). Other nucleic acids are not replicated by this amplification enzyme. Similarly, in the case of T7 RNA polymerase, this amplification enzyme has a stringent specificity for its own promoters (*See*, Chamberlin et al., Nature 228:227 [1970]). In the case of T4 DNA ligase, the enzyme will not ligate the two oligonucleotides or polynucleotides, where there is a mismatch between the oligonucleotide or polynucleotide substrate and the template at the ligation junction (*See*, Wu and Wallace, Genomics 4:560 [1989]). Finally, *Taq* and *Pfu* polymerases, by virtue of their ability to function at high temperature, are found to display high specificity for the sequences bounded and thus defined by the primers; the high temperature results in thermodynamic conditions that favor primer hybridization with the target sequences and not hybridization with non-target sequences.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (*i.e*., in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

As used herein, the term "probe" refers to an oligonucleotide (*i.e.*, a sequence of nucleotides), whether occurring naturally as in a purified restriction digest or produced synthetically, recombinantly or by PCR amplification, which is capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule," so that is detectable in any detection system, including, but not limited to enzyme (*e.g*., ELISA, as well as enzyme-based histochemical assays), fluorescent, radioactive, and luminescent systems. It is not intended that the present invention be limited to any particular detection system or label.

As used herein, the term "target," when used in reference to the polymerase chain reaction, refers to the region of nucleic acid bounded by the primers used for polymerase chain reaction. Thus, the "target" is sought to be sorted out from other nucleic acid sequences. A "segment" is defined as a region of nucleic acid within the target sequence.

As used herein, the term "polymerase chain reaction" ("PCR") refers to the methods of U.S. Patent Nos. 4,683,195 4,683,202, and 4,965,188, which include methods for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing and polymerase extension can be repeated many times (*i.e.*, denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified".

As used herein, the term "amplification reagents" refers to those reagents (deoxyribonucleotide triphosphates, buffer, etc.), needed for amplification except for primers, nucleic acid template and the amplification enzyme. Typically, amplification reagents along with other reaction components are placed and contained in a reaction vessel (test tube, microwell, etc.).

With PCR, it is possible to amplify a single copy of a specific target sequence in genomic DNA to a level detectable by several different methodologies (*e.g*., hybridization with a labeled probe; incorporation of biotinylated primers followed by avidin-enzyme conjugate detection; incorporation of ³²P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process itself are, themselves, efficient templates for subsequent PCR amplifications.

As used herein, the terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.

As used herein, the terms "restriction endonucleases" and "restriction enzymes" refer to bacterial enzymes, each of which cut double-stranded DNA at or near a specific nucleotide sequence.

As used herein, "personal care products" means products used in the cleaning of hair, skin, scalp, teeth, including, but not limited to shampoos, body lotions, shower gels, topical moisturizers, toothpaste, and/or other topical cleansers. In some particularly preferred embodiments, these products are utilized by humans, while in other embodiments, these products find use with non-human animals (*e.g*., in veterinary applications).

As used herein, "cleaning compositions" are compositions that can be used to remove undesired compounds from substrates, such as fabric, dishes, contact lenses, other solid substrates, hair (shampoos), skin (soaps and creams), teeth (mouthwashes, toothpastes) etc.

The term "cleaning composition materials," as used herein, refers to any liquid, solid or gaseous material selected for the particular type of cleaning composition desired and the form of the product (*e.g*., liquid; granule; spray composition), which materials are also compatible with the amylase and other enzyme(s) and used in the composition. The specific selection of cleaning composition materials are readily made by considering the surface, item or fabric to be cleaned, and the desired form of the composition for the cleaning conditions during use (*e.g.*, through the wash detergent use).

As used herein the term "hard surface cleaning composition," refers to detergent compositions for cleaning hard surfaces such as floors, walls, bathroom tile, and the like. Such compositions are provided in any form, including but not limited to solids, liquids, emulsions, etc.

As used herein, "dishwashing composition" refers to all forms for compositions for cleaning dishes, including but not limited to, granular and liquid forms.

As used herein, "fabric cleaning composition" refers to all forms of detergent compositions for cleaning fabrics, including but not limited to, granular, liquid and bar forms. As used herein, "fabric" refers to any textile material.

As used herein, the term "compatible," means that the cleaning composition materials do not reduce the proteolytic activity of the amylase to such an extent that the amylase is not effective as desired during normal use situations. Specific cleaning composition materials are exemplified in detail hereinafter.

As used herein, "effective amount of amylase enzyme" refers to the quantity of amylase enzyme necessary to achieve the enzymatic activity required in the specific application (*e.g*., personal care product, cleaning composition, etc.). Such effective amounts are readily ascertained by one of ordinary skill in the art and are based on many factors, such as the particular enzyme variant used, the cleaning application, the specific composition of the cleaning composition, and whether a liquid or dry (*e.g*., granular, bar) composition is required, and the like.

As used herein, "non-fabric cleaning compositions" encompass hard surface cleaning compositions, dishwashing compositions, oral cleaning compositions, denture cleaning compositions, and personal cleansing compositions.

As used herein, "oral cleaning compositions" refers to dentifrices, toothpastes, toothgels, toothpowders, mouthwashes, mouth sprays, mouth gels, chewing gums, lozenges, sachets, tablets, biogels, prophylaxis pastes, dental treatment solutions, and the like.

As used herein, "pharmaceutically-acceptable" means that drugs, medicaments and/or inert ingredients which the term describes are suitable for use in contact with the tissues of humans and other animals without undue toxicity, incompatibility, instability, irritation, allergic response, and the like, commensurate with a reasonable benefit/risk ratio.

Many of the protein variants of the present invention are useful in formulating various detergent compositions. A number of known compounds are suitable surfactants useful in compositions comprising the protein mutants of the invention. These include nonionic, anionic, cationic, anionic or zwitterionic detergents (*See e.g*., US Patent No 4,404,128 and US Patent No. 4,261,868). A suitable detergent formulation is that described in Example 7 of US Patent 5,204,015. Those in the art are familiar with the different formulations which find use as cleaning compositions. In addition to typical cleaning compositions, it is readily understood that the protein variants of the present invention find use in any purpose that native or wild-type proteins are used. Thus, these variants can be used, for example, in bar or liquid soap applications, dishcare formulations, surface cleaning applications, contact lens cleaning solutions or products, peptide hydrolysis, waste treatment, textile applications, as fusion-cleavage enzymes in protein production, etc. Indeed, it is not intended that the variants of the present invention be limited to any particular use. For example, the variants of the present invention may comprise, in addition to decreased allergenicity, enhanced performance in a detergent composition (as compared to the precursor). As used herein, "enhanced performance" in a detergent is defined as increasing cleaning of certain enzyme sensitive stains (*e.g*., grass or blood), as determined by usual evaluation after a standard wash cycle. In particular, the amylases of the present invention provide enhanced performance in the removal of starch-based stains and soils such as chocolate, gravy, baby food, etc., from clothes and other materials. In addition, the amylases provide enhanced performance in removing rice, pasta, oatmeal, etc., from dishes.

Proteins, particularly amylases, can be formulated into known powdered and liquid detergents having pH between 6.5 and 12.0 at levels of about .01 to about 5% (preferably 0.1% to 0.5%) by weight. These detergent cleaning compositions may further include other enzymes such as proteases, additional amylases, cellulases, lipases or endoglycosidases, as well as builders and stabilizers.

The addition of proteins to conventional cleaning compositions does not create any special use limitations. In other words, any temperature and pH suitable for the detergent are also suitable for the present compositions, as long as the pH is within the above range, and the temperature is below the described protein's denaturing temperature. In addition, proteins described herein find use in cleaning compositions without detergents, again either alone or in combination with builders and stabilizers.

Provided herein are compositions for the treatment of textiles that includes variant proteins of the present invention. The composition can be used to treat for example silk or wool (*See e.g*., RE 216,034; EP 134,267; US 4,533,359; and EP 344,259). As needed, these variants are screened for proteolytic activity according to methods well known in the art. In particular, provided herein are amylases suitable for fabric care and treatment applications, including but not limited to desizing.

The amylases description herein find use in industries such as food and feed production, baking, brewing, the pulp and paper industries, liquefaction and/or degradation of starch, modification of starch, grain processing and milling, maltogenesis, cereal starch liquefaction, wort production, all-barley brewing,

As indicated above, the amylases described herein exhibit modified immunogenic responses (*e.g*., antigenicity and/or immunogenicity) when compared to the native amylases encoded by their precursor DNAs. The proteins (*e.g*., amylases) preferably exhibit reduced allergenicity/immunogenicity. Those of skill in the art readily recognize that the uses of the amylases will be determined, in large part, on the immunological properties of the proteins. For example, amylases that exhibit reduced immunogenic responses can be used in cleaning compositions. An effective amount of one or more amylase variants described herein find use in compositions useful for cleaning a variety of surfaces in need of starchy stain removal. Such cleaning compositions include detergent compositions for cleaning hard surfaces, detergent compositions for cleaning fabrics, dishwashing compositions, oral cleaning compositions, and denture cleaning compositions.

The amylases find further use in formulations designed to release their active ingredients upon contact with moisture. Thus, starch-based compositions may be rapidly broken down upon contact with the amylase(s) under moist conditions. In addition, the amylases find use as digestive aids (*e.g*., for starch metabolism).

The compositions described herein may contain an emulsifier and/or surfactant, generally to help disperse and suspend the disperse phase within the continuous aqueous phase. A surfactant may also be useful if the product is intended for skin cleansing. For convenience hereinafter, emulsifiers are encompassed within the term "surfactants." Thus, "surfactant(s)" refer(s) to surface active agents whether used as emulsifiers or for other surfactant purposes such as skin cleansing. Known or conventional surfactants find use used in the compositions, provided that the selected agent is chemically and physically compatible with essential components of the composition, and provides the desired characteristics. Suitable surfactants include non-silicone derived materials, and mixtures thereof. All surfactants discussed in application WO 00/24372 are considered as suitable.

The compositions comprise from about 0.05% to about 15% of a surfactant or mixture of surfactants. The exact surfactant or surfactant mixture chosen will depend upon the pH of the composition and the other components present.

Among the nonionic surfactants that are useful herein are those that can be broadly defined as condensation products of long chain alcohols (*e.g.* C₈₋₃₀ alcohols), with sugar or starch polymers (*i.e.*, glycosides). Other useful nonionic surfactants include the condensation products of alkylene oxides with fatty acids (*i.e.*, alkylene oxide esters of fatty acids). These materials have the general formula RCO(X)ₙOH wherein R is a C₁₀₋₃₀ alkyl group, X is -OCH₂CH₂- (*i.e.*, derived from ethylene glycol or oxide) or -OCH₂CHCH₃- (*i.e.*, derived from propylene glycol or oxide), and n is an integer from about 6 to about 200. Other nonionic surfactants are the condensation products of alkylene oxides with 2 moles of fatty acids (*i.e.*, alkylene oxide diesters of fatty acids). These materials have the general formula RCO(X)ₙOOCR wherein R is a C₁₀₋₃₀ alkyl group, X is -OCH₂CH₂-(*i.e.* derived from ethylene glycol or oxide) or -OCH₂CHCH₃-(*i.e.*, derived from propylene glycol or oxide), and n is an integer from about 6 to about 100. An emulsifier for use herein is most preferably a fatty acid ester blend based on a mixture of sorbitan fatty acid ester and sucrose fatty acid ester, especially a blend of sorbiton stearate and sucrose cocoate. This is commercially available from ICI under the trade name Arlatone 2121. Even further suitable examples include a mixture of cetearyl alcohols, cetearyl glucosides such as those available under the trade name Montanov 68 from Seppic and Emulgade PL68/50 available from Henkel.

The hydrophilic surfactants useful herein may alternatively or additionally include any of a wide variety of cationic, anionic, zwitterionic, and amphoteric surfactants such as are known in the art (*See e.g*., US Patent No. 5,011,681, US Patent No. 4,421,769, and US Patent No. 3,755,560). A wide variety of anionic surfactants also find use in the compositions of the present invention (*See e.g*., US Patent No. 3,929,678). Exemplary anionic surfactants include the alkoyl isethionates (*e.g*., C₁₂-C₃₀), alkyl and alkyl ether sulfates and salts thereof, alkyl and alkyl ether phosphates and salts thereof, alkyl methyl taurates (*e.g*., C₁₂ - C₃₀), and soaps (*e.g*., alkali metal salts, such as sodium or potassium salts) of fatty acids.

Amphoteric and zwitterionic surfactants also find use in the compositions of the present invention. Examples of amphoteric and zwitterionic surfactants which can be used in the compositions of the present invention are those which are broadly described as derivatives of aliphatic secondary and tertiary amines in which the aliphatic radical can be straight or branched chain and wherein one of the aliphatic substituents contains from about 8 to about 22 carbon atoms (preferably C₈ - C₁₈) and one contains an anionic water solubilizing group (*e.g*., carboxy, sulfonate, sulfate, phosphate, or phosphonate). Examples include alkyl imino acetates, iminodialkanoates and aminoalkanoates, imidazolinium and ammonium derivatives. Other suitable amphoteric and zwitterionic surfactants include those selected from the group consisting of betaines, sultaines, hydroxysultaines, and branched and unbranched alkanoyl sarcosinates, and mixtures thereof.

Emulsions may further include a silicone containing emulsifier or surfactant. A wide variety of silicone emulsifiers find use in the present invention. These silicone emulsifiers are typically organically modified organopolysiloxanes, also known to those skilled in the art as silicone surfactants. Useful silicone emulsifiers include dimethicone copolyols. These materials are polydimethyl siloxanes which have been modified to include polyether side chains such as polyethylene oxide chains, polypropylene oxide chains, mixtures of these chains, and polyether chains containing moieties derived from both ethylene oxide and propylene oxide. Other examples include alkyl-modified dimethicone copolyols (*i.e.*, compounds which contain C₂-C₃₀ pendant side chains). Still other useful dimethicone copolyols include materials having *various* cationic, anionic, amphoteric, and zwitterionic pendant moieties.

The compositions described herein may comprise at least one polymeric thickening agent. The polymeric thickening agents useful herein preferably have a number average molecular weight of greater than 20,000, more preferably greater than 50,000 and especially greater than 100,000. The compositions may comprise from about 0.01 % to about 10%, preferably from about 0.1 % to about 8% and most preferably from about 0.5% to about 5% by weight of the composition of the polymeric thickening agent, or mixtures thereof.

Preferred polymer thickening agents for use herein include non-ionic thickening agents and anionic thickening agents, or mixtures thereof. Suitable non-ionic thickening agents include polyacrylamide polymers, crosslinked poly(N-vinylpyrrolidones), polysaccharides, natural or synthetic gums, polyvinylpyrrolidone, and polyvinylalcohol. Suitable anionic thickening agents include acrylic acid/ethyl acrylate copolymers, carboxyvinyl polymers and crosslinked copolymers of alkyl vinyl ethers and maleic anhydride. Particularly preferred thickening agents for use herein are the non-ionic polyacrylamide polymers such as polyacrylamide and isoparaffin and laureth-7, available under the trade name Sepigel 305 from Seppic Corporation, and acrylic acid/ethyl acrylate copolymers and the carboxyvinyl polymers sold by the B.F. Goodrich Company under the trade mark of CARBOPOL™ resins, or mixtures thereof. Suitable CARBOPOL™ resins may be hydrophobically modified. Additional suitable resins are described in WO98/22085. Mixtures of these resins are also contemplated.

Compositions may comprise, at least one silicone oil phase. Silicone oil phase(s) generally comprises from about 0.1 % to about 20%, preferably from about 0.5% to about 10%, more preferably from about 0.5% to about 5%, of the composition. The, or each, silicone oil phase preferably comprises one or more silicone components.

Silicone components are fluids, including straight chain, branched and cyclic silicones. Suitable silicone fluids useful herein include silicones inclusive of polyalkyl siloxane fluids, polyaryl siloxane fluids, cyclic and linear polyalkylsiloxanes, polyalkoxylated silicones, amino and quaternary ammonium modified silicones, polyalkylaryl siloxanes or a polyether siloxane copolymer and mixtures thereof. The silicone fluids can be volatile or non-volatile. Silicone fluids generally have a weight average molecular weight of less than about 200,000. Suitable silicone fluids have a molecular weight of about 100,000 or less, preferably about 50,000 or less, most preferably about 10,000 or less. Preferably the silicone fluid is selected from silicone fluids having a weight average molecular weight in the range from about 100 to about 50,000 and preferably from about 200 to about 40,000. Typically, silicone fluids have a viscosity ranging from about 0.65 to about 600,000 mm².s⁻¹, preferably from about 0.65 to about 10,000 mm².s⁻¹ at 25°C. The viscosity can be measured by means of a glass capillary viscometer as set forth in Dow Corning Corporate Test Method CTM0004. Suitable polydimethyl siloxanes include those available, for example, from the General Electric Company as the SF and Viscasil (RTM) series and from Dow Corning as the Dow Corning 200 series. Also useful are essentially non-volatile polyalkylarylsiloxanes (*e.g*., polymethylphenylsiloxanes), having viscosities of about 0.65 to 30,000 mm².s⁻¹ at 25°C. These siloxanes are available, for example, from the General Electric Company as SF 1075 methyl phenyl fluid or from Dow Corning as 556 Cosmetic Grade Fluid. Cyclic polydimethylsiloxanes suitable for use herein are those having a ring structure incorporating from about 3 to about 7 (CH₃)₂SiO moieties.

Silicone gums also find use. The term "silicone gum" herein means high molecular weight silicones having a weight average molecular weight in excess of about 200,000 and preferably from about 200,000 to about 4,000,000. Non-volatile polyalkyl as well as polyaryl siloxane gums are contemplated A silicone oil phase may comprise a silicone gum or a mixture of silicones including the silicone gum. Typically, silicone gums have a viscosity at 25°C in excess of about 1,000,000 mm²s⁻¹. The silicone gums include dimethicones as known in the art (*See e.g*., US Patent No. 4,152,416), as well as the silicone gums described in General Electric Silicone Rubber Product Data Sheets SE 30, SE 33, SE 54 and SE 76. Specific examples of silicone gums include polydimethylsiloxane, (polydimethylsiloxane)-(methylvinylsiloxane) copolymer, poly(dimethylsiloxane)(diphenyl)(methylvinylsiloxane) copolymer and mixtures thereof. Preferred silicone gums for use herein are silicone gums having a molecular weight of from about 200,000 to about 4,000,000 selected from dimethiconol, dimethicone copolyol, dimethicone, and mixtures thereof.

A silicone phase herein preferably comprises a silicone gum incorporated into the composition as part of a silicone gum-fluid blend. When the silicone gum is incorporated as part of a silicone gum-fluid blend, the silicone gum preferably constitutes from about 5% to about 40%, especially from about 10% to 20% by weight of the silicone gum-fluid blend. Suitable silicone gum-fluid blends herein are mixtures consisting essentially of:
(i) a silicone having a molecular weight of from about 200,000 to about 4,000,000 selected from dimethiconol, fluorosilicone and dimethicone and mixtures thereof; and
(ii) a carrier which is a silicone fluid, the carrier having a viscosity from about 0.65 mm².s⁻¹ to about 100 mm².s⁻¹,
wherein the ratio of i) to ii) is from about 10:90 to about 20:80 and wherein the silicone gum-based component has a final viscosity of from about 100 mm².s⁻¹ to about 100,000 mm².s⁻¹, preferably from 500 mm².s⁻¹ to about 10,000 mm².s⁻¹.

Further silicone components suitable for use in a silicone oil phase herein are crosslinked polyorganosiloxane polymers, optionally dispersed in a fluid carrier. In general, crosslinked polyorganosiloxane polymers, together with its carrier (if present) comprise 0.1% to about 20%, preferably from about 0.5% to about 10%, more preferably from about 0.5% to about 5% of the composition. Such polymers comprise polyorganosiloxane polymers crosslinked by a crosslinking agent. Suitable crosslinking agents include those described in WO98/22085. Examples of suitable polyorganosiloxane polymers for use herein include methyl vinyl dimethicone, methyl vinyl diphenyl dimethicone, and methyl vinyl phenyl methyl diphenyl dimethicone.

Another class of silicone components suitable for use in a silicone oil phase herein includes polydiorganosiloxane-polyoxyalkylene copolymers containing at least one polydiorganosiloxane segment and at least one polyoxyalkylene segment. Suitable polydiorganosiloxane segments and copolymers thereof include those described in WO98/22085. Suitable polydiorganosiloxane-polyalkylene copolymers are available commercially under the trade names Belsil (RTM) from Wacker-Chemie GmbH, Munich, and Abil (RTM) from Th. Goldschmidt Ltd., England, for example Belsil (RTM) 6031 and Abil (RTM) B88183. A particularly preferred copolymer fluid blend for use herein includes Dow Corning DC3225C which has the CTFA designation Dimethicone/Dimethicone copolyol.

The compositions described herein may comprise antimicrobial and/or antifungal actives. Non-limiting examples of antimicrobial and antifungal actives useful herein include, but are not limited to ß-lactam drugs, quinolone drugs, ciprofloxacin, norfloxacin, tetracycline, erythromycin, amikacin, 2,4,4'-trichloro-2'-hydroxy diphenyl ether, 3,4,4'-trichlorobanilide, phenoxyethanol, phenoxy propanol, phenoxyisopropanol, doxycycline, capreomycin, chlorhexidine, chlortetracycline, oxytetracycline, clindamycin, ethambutol, hexamidirie isethionate, metronidazole, pentamidine, gentamicin, kanamycin, lineomycin, methacycline, methenamine, minocycline, neomycin, netilmicin, paromomycin, streptomycin, tobramycin, miconazole, tetracycline hydrochloride, erythromycin, zinc erythromycin, erythromycin estolate, erythromycin stearate, amikacin sulfate, doxycycline hydrochloride, capreomycin sulfate, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, amanfadine hydrochloride, amanfadine sulfate, octopirox, parachlorometa xylenol, nystatin, tolnaftate, clotrimazole, cetylpyridinium chloride (CPC), piroctone olamine, selenium sulfide, ketoconazole, triclocarbon, triclosan, zinc pyrithione, itraconazole, asiatic acid, hinokitiol, mipirocin, clinacycin hydrochloride, benzoyl peroxide, benzyl peroxide, minocyclin, phenoxy isopropanol, and mixtures thereof, as well as those described in European Patent No. 0 680 745.

A variety of optional ingredients such as neutralizing agents, perfumes, and coloring agents, find use in the compositions. In addition it is preferred that any such ingredients do not negatively impact the aesthetic properties of the product.

Neutralizing agents suitable for use in neutralizing acidic group containing hydrophilic gelling agents herein include sodium hydroxide, potassium hydroxide, ammonium hydroxide, monoethanolamine, triethanolamine, amino methyl propanol, tris-buffer and triethanolamine.

Other optional materials herein include pigments which, when water-insoluble, contribute to and are included in the total level of oil phase ingredients. Pigments suitable for use in the compositions can be organic and/or inorganic. Also included within the term "pigment" are materials having a low colour or luster such as matte finishing agents, and also light scattering agents. Preferably, the compositions of the present invention comprise particulate materials having a refractive index of from about 1.3 to about 1.7, the particulate materials being dispersed in the composition and having a median particle size of from about 2 to about 30 µm. Preferably the particulates useful herein have relatively narrow distributions, by which is meant that more than 50% of the particles fall within 3 µm either side of the respective median value. It is also preferred that more than 50%, preferably more than 60%, and even more preferably more than 70% of particles fall within the size ranges prescribed for the respective median values. Suitable particulate materials include organic or organosilicone and preferably organosilicone polymers. Preferred particles are free-flowing, solid, materials. By "solid" is meant that the particles are not hollow. The void at the center of hollow particles can have an adverse effect on refractive index and therefore the visual effects of the particles on either skin or the composition. Suitable organic particulate materials include those made of polymethylsilsesquioxane, referenced above, polyamide, polythene, polyacrylonitrile, polyacrylic acid, polymethacrylic acid, polystyrene, polytetrafluoroethylene (PTFE) and poly(vinylidene chloride). Copolymers derived from monomers of the aforementioned materials can also be used. Inorganic materials include silica and boron nitride. Representative commercially available examples of useful particulate materials herein are Tospearl^{®} 145 which has a median particle size of about 4.5 µm and EA-209^{®} from Kobo which is an ethylene / acrylic acid copolymer having a median particle size of about 10 µm, Nylon-12 available under the trade name Orgasol 2002 from Elf Atochem, France, or mixtures thereof.

Further examples of suitable pigments include titanium dioxide, predispersed titanium dioxide from Kobo (*e.g*., Kobo GWL75CAP), iron oxides, acyglutamate iron oxides, ultramarine blue, D&C dyes, carmine, and mixtures thereof. Depending upon the type of composition, a mixture of pigments will often find use. The preferred pigments for use herein from the viewpoint of moisturisation, skin feel, skin appearance and emulsion. Suitably, the pH of the compositions herein is in the range from about 6.1 to about 10.0, wherein the pH of the final composition is adjusted by addition of acidic, basic or buffer salts as necessary.

The compositions are prepared by standard techniques well known to those skilled in the art. In general, the aqueous phase and/ or the oil phase are prepared separately, with materials of similar phase partitioning being added in any order. If the final product is an emulsion, the two phases are then combined with vigorous stirring. Any ingredients in the formulation with high volatility, or which are susceptible to hydrolysis at high temperatures, can be added with gentle stirring towards the end of the process, post emulsification if applicable.

As indicated above, amylases with reduced allergenicity/immunogenicity also find use in the treatment of textiles. "Textile treatment" comprises a process wherein textiles, individual yarns or fibers that can be woven, felted or knitted into textiles or garments are treated to produce a desired characteristic. Examples of such desired characteristics are "stone-washing," depilling, dehairing, desizing, softening, and other textile treatments well known to those of skill in the art.

The epitopes identified herein may be used to elicit an immune response (*e.g*., where it is desired to raise antibodies against an amylase including one or both of such epitopes. Such antibodies find use in screening for other amylases that include one or both of these regions, or regions highly homologous thereto. In addition, the amylases find use as reagents in various assays, such as immunoassays utilizing isolated natural epitope, recombinant protein, or synthetic peptide representing specific epitopic regions to evaluate persons for sensitization to proteins including these or highly homologous regions.

The eptiopic fragments identified herein may be used in the detection of antigen presenting cells having MHC molecules capable of binding and displaying such fragments. For example, the epitopic fragments can include a detectable label (*e.g*., radiolabel). The labeled fragments are then incubated with cells of interest, and then cells which bind (or display) the labeled fragments are detected.

The related and/or variant amylases with reduced allergenicity/immunogenicity may find use in other applications, including pharmaceutical applications, drug delivery applications, and other health care applications. In addition, the related and/or variant proteins with reduced allergenicity/immunogenicity find use in other applications, including pharmaceutical applications, drug delivery applications, and other health care applications. Indeed, it is contemplated that the amylases will find widespread use in numerous compositions and applications.

### DETAILED DESCRIPTION OF THE INVENTION

The present invention provides methods for reducing immunogenicity of a precursor microbial amylase as defined in the claims.

As described herein, there are at least four T-cell epitopes in the amylases analyzed as described herein. Indeed, the following epitopes were determined to be of interest: DSAYLAEHGITAVWIPPAYKG (SEQ ID NO:2), KYGTKGELQSAIKSL (SEQ ID NO:3), DRNRVISGEHLIKAW (SEQ ID NO:4), FDGTDWDESRKLNRI (SEQ ID NO:5), AWDWEVSNENGNYDY (SEQ ID NO:6), EIKRWGTWYANELQL (SEQ ID NO:7), EPILKARKQYAYGAQ (SEQ ID NO:8), RQNAGETWHDITGNR (SEQ ID NO:9), and EFHVNGGSVSIYVQR (SEQ ID NO:10).

The methods of the invention comprise modifying a precursor microbial amylase at an epitope equivalent to SEQ ID NO: 5 of Bacillus lichenformis amylase, as defined in the claims.

Typically, at least one amino acid substitution is made at least one T-cell epitope in the wild-type or parental amylase sequence. In some preferred embodiments, multiple amino acids are changed in the parental amylase sequence to produce an amylase variant having reduced allergenicity/immunogenicity. In alternative preferred embodiments, amino acid deletions, insertions and/or substitutions are made in the parental amylase sequence to produce a variant amylase with reduced allergenicity/immunogenicity. In some embodiments, the amylase is *B. licheniformis* amylase, while in alternative embodiments, the amylase is an amylase obtained from any other organism.. In some embodiments, this amylase is wild-type, while in other embodiments, it is a mutated variant, conjugated variant, or a hybrid variant having amino acid substitutions in the epitope of interest, which can cause sensitization in an individual or sampling of individuals.

A peptide having an altered immunogenic response, (e.g., increased or decreased immunogenic response may be derived from an amylase of interest. The epitope may be identified by an assay which identifies epitopes and non-epitopes in which differentiated dendritic cells are combined with naive human CD4+ and/or CD8+ T-cells and with a peptide of interest.

Typically, a series of peptide oligomers which correspond to all or part of the amylase of interest are prepared, e.g. a peptide library is produced covering the relevant portion or all of the protein. As described in the Examples below, a set of 15-mer peptides offset by three amino acids was used to identify the epitopes of interest. By analyzing each of the peptides individually in the assay provided herein, the methods of the present invention facilitate the precise identification of epitopes recognized by T-cells. In the example above, the greater reaction of one specific peptide than its neighbors' facilitates identification of the epitope anchor region to within three amino acids. After the locations of these epitopes are determined, one or more of the amino acids within each epitope may be modified, until the peptide produces a different T-cell response from that of the original protein. In the methods of the present invention, an epitope equivalent to SEQ ID NO: 5 is modified.

Amylases are preferably isolated or purified. By "purification or isolation" it is meant that the amylase is altered from its natural state by virtue of separating the amylase from some or all of the naturally occurring constituents with which it is associated in nature. It is intended that this purification be accomplished by any suitable means known in the art, including but not limited to art-recognized separation techniques such as ion exchange chromatography, affinity chromatography, hydrophobic separation, dialysis, amylase treatment, ammonium sulfate precipitation or other protein salt precipitation, centrifugation, size exclusion chromatography, filtration, microfiltration, gel electrophoresis or separation on a gradient to remove whole cells, cell debris, impurities, extraneous proteins, and/or enzymes undesired in the final composition. In some embodiments, constituents are added to the amylase-containing composition to provide additional benefits, for example, activating agents, anti-inhibition agents, desirable ions, compounds to control pH, and/or other enzymes (*e.g*., cellulase).

Exemplary assays useful in ascertaining the reduced immunogenic response of the variant proteins include, but are not limited to *in vivo* assays (HLA-DR3/DQ2 mouse T cell responses, and *in vitro* assays (human peripheral blood mononuclear cells (PBMC) to Amylase 1 (*i.e*., a BPN'-Y217L amylase) and its variants. *In vivo* assays useful in ascertaining the reduced immunogenic response include, but are not limited to the use of transgenic mice, for example, rats (Taurog et al., Immunol. Rev., 169:209-223 [1999]), rabbits, or pigs. A preferred transgenic mouse model for testing modified proteins of interest and variants *in vivo*, determining a reduced immunogenic response, is the HLA-DR3/DQ2 mouse model known in the art. The amylase epitopes of interest were shown to bind to HLA-DQ2 by cell surface binding analyses.

In addition to decreasing the immunogenic response of an animal, such as a human, to naturally occurring amino acid sequences, the present invention provides means for reducing the immunogenic response of mutated proteins (*e.g*., an amylase that has been altered to change the functional activity of the protein). In some embodiments, the mutation is made in order to provide some beneficial characteristic, including but not limited to increased activity, increased thermal stability, increased alkaline stability and/or oxidative stability. In some embodiments, the mutation results in the incorporation of new T-cell epitope in the mutated amylase. As discussed in greater detail herein Amylase T-cell epitopes and variant amylases are described with amino acid alterations that will alter the immunogenic response of the mutated protein.

The following describes particularly preferred embodiments of the invention, which involve the modification of amylase.

The amino acid position numbers used herein refer to those assigned to the mature *Bacillus licheniformis* amylase sequence of SEQ ID NO:1. The invention, however, is not limited to the mutation of this particular amylase but extends to precursor amylases containing amino acid residues at positions which are "equivalent" to the particular identified residues in *Bacillus licheniformis* amylase. For example, where the precursor amylase is *Bacillus licheniformis* alpha-amylase, substitutions, deletions and/or insertions can be made at the equivalent amino acid residue in *B.licheniformis* corresponding to those listed above.

A residue (amino acid) of a precursor amylase is equivalent to a residue of *Bacillus licheniformis* amylase if it is either homologous (*i.e*., corresponding in position in either primary or tertiary structure) or analogous to a specific residue or portion of that residue in *Bacillus licheniformis* amylase (*i.e*., having the same or similar functional capacity to combine, react, or interact chemically). "Corresponding," as used herein generally refers to an analogous position along the peptide.

In order to establish homology to primary structure, the amino acid sequence of a precursor amylase is directly compared to the *Bacillus licheniformis* amylase primary sequence and particularly to a set of residues known to be invariant in amylases for which the sequence is known. After aligning the conserved residues, allowing for necessary insertions and deletions in order to maintain alignment (*i.e*., avoiding the elimination of conserved residues through arbitrary deletion and insertion), the residues equivalent to particular amino acids in the primary sequence of *Bacillus licheniformis* amylase are defined. Alignment of conserved residues preferably should conserve 100% of such residues. However, alignment of greater than 75% or as little as 50% of conserved residues is also adequate to define equivalent residues. These conserved residues, thus, may be used to define the corresponding equivalent amino acid residues of *Bacillus licheniformis* amylase in other amylases which are highly homologous to the preferred *Bacillus licheniformis* amylase.

While the instant invention is useful to lower the immunogenic response, the mutations specified herein find use in combination with mutations known in the art to result altered thermal stability and/or altered substrate specificity, modified activity, improved specific activity or altered alkaline stability as compared to the precursor.

Two homologous proteins may be fused so as to eliminate at least one T-cell epitope. As is described below, a region of a protein in which a T-cell epitope resides can be replaced with the same region in a homologous protein that does not have the T-cell epitope. For example, a fusion protein is created with amylase from *Bacillus licheniformis* and its *B*. *licheniformis* homolog, so that the resulting protein does not have the T-cell epitope present in the parental *B. licheniformis* amylase. Any amino acid length can be used for fusion into the parental protein, from only the epitope to the majority of the protein, as long as the desired activity is maintained. However, it is not necessary that the original level of activity be maintained. Because of the lowered allergenicity/immunogenicity of the protein, it may be possible to use more of the hybrid protein than the parental protein, in order to achieve the same activity levels.

The variant amylase activity may be determined and compared with the amylase of interest by examining the interaction of the amylase with various commercial substrates, including, but not limited to casein, keratin, elastin, collagen. As desired, amylase activity can be determined by any suitable method known to those in the art. In additional embodiments, the other characterisitics of the variant amylases are determined using suitable methods known to those in the art. Exemplary characteristics include, but are not limited to thermal stability, alkaline stability, and stability of the particular amylase in various substrate or buffer solutions or product formulations. When combined with the enzyme stability assay procedure disclosed herein, mutants obtained by random mutagenesis are identified which demonstrated either increased or decreased alkaline or thermal stability while maintaining enzymatic activity.

### EXPERIMENTAL

The following examples serve to illustrate certain preferred embodiments and aspects of the present invention and are not to be construed as limiting the scope thereof.

In the experimental disclosure which follows, the following abbreviations apply: M (molar); mM (millimolar); µM (micromolar); nM (nanomolar); mol (moles); mmol (millimoles); µmol (micromoles); nmol (nanomoles); gm (grams); mg (milligrams); µg (micrograms); pg (picograms); L (liters); ml (milliliters); µl (microliters); cm (centimeters); mm (millimeters); µm (micrometers); nm (nanometers); °C (degrees Centigrade); cDNA (copy or complimentary DNA); DNA (deoxyribonucleic acid); ssDNA (single stranded DNA); dsDNA (double stranded DNA); dNTP (deoxyribonucleotide triphosphate); RNA (ribonucleic acid); PBS (phosphate buffered saline); *g* (gravity); OD (optical density); Dulbecco's phosphate buffered solution (DPBS); HEPES (N-[2-Hydroxyethyl]piperazine-N-[2-ethanesulfonic acid]); HBS (HEPES buffered saline); SDS (sodium dodecylsulfate); Tris-HCl (tris[Hydroxymethyl]aminomethane-hydrochloride); Klenow (DNA polymerase I large (Klenow) fragment); rpm (revolutions per minute); EGTA (ethylene glycol-bis(ß-aminoethyl ether) N, N, N', N'-tetraacetic acid); EDTA (ethylenediaminetetracetic acid); bla (ß-lactamase or ampicillin-resistance gene); Endogen (Endogen, Woburn, MA); CytoVax (CytoVax, Edmonton, Canada); Wyeth-Ayerst (Wyeth-Ayerst, Philadelphia, PA); NEN (NEN Life Science Products, Boston, MA); Wallace Oy (Wallace Oy, Turku, Finland); Pharma AS (Pharma AS, Oslo, Norway); Dynal (Dynal, Oslo, Norway); Bio-Synthesis (Bio-Synthesis, Lewisville, TX); ATCC (American Type Culture Collection, Rockville, MD); Gibco/BRL (Gibco/BRL, Grand Island, NY); Sigma (Sigma Chemical Co., St. Louis, MO); Pharmacia (Pharmacia Biotech, Pisacataway, NJ); and Stratagene (Stratagene, La Jolla, CA).

### EXAMPLE 1

### Preparation of Cells Used in the I-MUNE® Assay System for the Identification of Peptide T-Cell Epitopes in Alpha Amylase Using Human T-Cells

Fresh human peripheral blood cells were collected from 82 humans of unknown exposure status to IFN-β. These cells were tested to determine antigenic epitopes in IFN-β, as described in Example 3.

Peripheral mononuclear blood cells (stored at room temperature, no older than 24 hours) were prepared for use as follows: Approximately 30 mls of a solution of buffy coat preparation from one unit of whole blood was brought to 50 ml with Dulbecco's phosphate buffered solution (DPBS) and split into two tubes. The samples were underlaid with 12.5 ml of room temperature Lymphoprep density separation media (Nycomed; Pharma AS; Density 1.077 g/ml). The tubes were centrifuged for thirty minutes at 600 xg. The interface of the two phases was collected, pooled and washed in DPBS. The cell density of the resultant solution was measured by hemocytometer, as known in the art. Viability was measured by trypan blue exclusion, as known in the art.

From the resulting solution, a differentiated dendritic cell culture was prepared from the peripheral blood mononuclear cell sample having a density of 10⁸ cells per 75 ml culture flask in a solution as described below:
(1) 50 ml of serum free AIM V media (Gibco) was supplemented with a 1:100 dilution beta-mercaptoethanol (Gibco). The flasks were laid flat for two hours at 37°C in 5% CO₂ to allow adherence of monocytes to the flask wall.
(2) Differentiation of the monocyte cells to dendritic cells was performed as follows: nonadherent cells were removed and the resultant adherent cells (monocytes) combined with 30 ml of AIM V, 800 units/ml of GM-CSF (Endogen) and 500 units/ml of IL-4 (Endogen); the resulting mixture was cultured for 5 days at 37°C in 5% CO₂. After the five days of incubation, the cytokine TNFα (Endogen) was added to 0.2 units/ml, and the cytokine IL-1α (Endogen) was added to a final concentration of 50 units/ml and the mixture incubated at 37°C in 5% CO₂ for two more days.
(3) On the seventh day, mitomycin C was added to a concentration of 50 micrograms/ml in 100 mM EDTA-containing PBS to stop growth of the now differentiated dendritic cell culture. The solution was incubated for 60 minutes at 37°C in 5% CO₂. Dendritic cells were dislodged from the plastic surface by gently rapping the flask. Dendritic cells were then centrifuged at 600 xg for 5 minutes, washed in DPBS and counted as described above.
(4) The prepared dendritic cells were placed into a 96 well round bottom plate at a concentration of 2x10⁴ cells/well in 100 microliter total volume of AIM V media, per well.

CD4+ T cells were prepared from frozen aliquots of the peripheral blood cell samples used to prepare the dendritic cells, using reagents provided by the Dynal CD4+ T-cell enrichment kit (Dynal). The resultant CD4+ cell solution was centrifuged, resuspended in AIMV media and the cell density was determined using methods known in the art. The CD4+ T-cell suspension was then resuspended to a count of 2x10⁶/ml in AIM V media to facilitate efficient manipulation of 96-well plates.

### EXAMPLE 2

### Identification of T-Cell Epitopes in Alpha Amylase

Peptides for use in the I-MUNE® assay described in Example 3 were prepared based on the sequence of *B*. *licheniformis* alpha amylase (Genbank P6278) with the sequence:

mkqqkrlyar lltllfalif llphsaaaaa nlngtlmqyf ewympndgqh wkrlqndsay
laehgitavw ippaykgtsq advgygaydl ydlgefhqkg tvrtkygtkg elqsaikslh
srdinvygdv vinhkggada tedvtavevd padrnrvisg ehrikawthf hfpgrgstys
dfkwhwyhfd gtdwdesrkl nriykfqgka wdwevsneng nydylmyadi dydhpdvaae
ikrwgtwyan elqldgfrld avkhikfsfl rdwvnhvrek tgkemftvae ywqndlgale
nylnktnfnh svfdvplhyq fhaastqggg ydmrkllnst vvskhplkav tfvdnhdtqp
gqslestvqt wfkplayafi ltresgypqv fygdmygtkg dsqreipalk hkiepilkar
kqyaygaqhd yfdhhdivgw tregdssvan sglaalitdg pggakrmyvg rqnagetwhd
itgnrsepvv insegwgefh vnggsvsiyv qr (SEQ ID NO:1).

Based upon the full length amino acid sequence (SEQ ID NO:1) of this α-amylase, a set of 157 15mers off-set by three amino acids comprising the entire sequence of α-amylase were synthetically prepared by Mimotopes.

Peptide antigen was prepared as a 2 mg/ml stock solution in DMSO. First, 0.5 microliters of the stock solution were placed in each well of the 96 well plate in which the differentiated dendritic cells were previously placed. Then, 100 microliters of the diluted CD4+ T-cell solution as prepared above, were added to each well. Useful controls include diluted DMSO blanks, and tetanus toxoid positive controls.

The final concentrations in each well, at 20 microliter total volume are as follows:
2x10⁴ CD4+
2x10⁵ dendritic cells (R:S of 10:1)
5 µM peptide

### EXAMPLE 3

### I-MUNE® Assay for the Identification of Peptide T-Cell Epitopes in Alpha Amylase Using Human T-Cells

Once the assay reagents (*i.e*., cells, peptides, etc.) were prepared and distributed into the 96-well plates, the I-MUNE® assays were conducted. Controls included dendritic cells plus CD4+ T-cells alone (with DMSO carrier) and with tetanus toxoid (Wyeth-Ayerst), at approximately 5 Lf/mL.

Cultures were incubated at 37°C in 5% CO₂ for 5 days. Tritiated thymidine (NEN) was added at 0.5 microCi/well. The cultures were harvested and assessed for incorporation the next day, using the Wallac TriBeta scintillation detection system (Wallace Oy).

All tests were performed at least in duplicate. All tests reported displayed robust positive control responses to the antigen tetanus toxoid. Responses were averaged within each experiment, then normalized to the baseline response. A positive event (*i.e*., a proliferative response) was recorded if the response was at least 2.95 times the baseline response.

The immunogenic responses (*i.e*., T-cell proliferation) to the prepared peptides from α-amylase were tallied and are shown in Figure 1. The overall background rate of responses to this peptide set was 2.80 ± 3.70%, for the donors tested. Using these methods various peptides of potential interest were identified, including those in Table 1, below.

**Table 1. Peptides of Interest in Alpha Amylase**

| **Peptide #** | **Sequence** | **SEQ ID NO:** |
|---|---|---|
| 10-12 | DSAYLAEHGITAVWIPPAYKG | 2 |
| 26 | KYGTKGELQSAIKSL | 3 |
| 42 | DRNRVISGEHLIKAW | 4 |
| 54 | FDGTDWDESRKLNRI | 5 |
| 61 | AWDWEVSNENGNYDY | 6 |
| 71 | EIKRWGTWYANELQL | 7 |
| 129 | EPILKARKQYAYGAQ | 8 |
| 148 | RQNAGETWHDITGNR | 9 |
| 157 | EFHVNGGSVSIYVQR | 10 |

It is contemplated that amino acid modifications in or around these peptides will provide variant α-amylases suitable for use as hypo-allergenic/immunogenic amylases.

### EXAMPLE 4

### HLA Association with an Epitope Peptide Number

The HLA-DR and DQ expression of all the donors tested in both rounds of assay testing described above are assessed using a commercially available PCR-based HLA typing kit (Bio-Synthesis). In some embodiments, the phenotypic frequencies of individual HLA-DR and -DQ antigens among responders and non-responders to a peptide number are tested using a chi-squared analysis with 1 degree of freedom. The increased or decreased likelihood of reacting to an epitope corresponding to the peptide number is calculated wherever the HLA antigen in question is present in both responding and non-responding donor samples and the corresponding epitope is considered an HLA associated epitope.

The magnitude of the proliferative response to an individual peptide in responders and non-responders expressing epitope-associated HLA alleles may also be analyzed. An "individual responder to the peptide" is defined by a stimulation index of greater than 2.95. It is contemplated that the proliferative response in donors who express an epitope associated with HLA alleles would be higher than in peptide responders who do not express the associated allele.

From the above, it is clear that the present invention provides methods and compositions for the identification of T-cell epitopes in wild-type amylase. Once antigenic epitopes are identified, the epitopes are modified as desired, and the peptide sequences of the modified epitopes incorporated into a wild-type amylase, so that the modified sequence is no longer capable of initiating the CD4⁺T-cell response or wherein the CD4⁺T -cell response is significantly reduced in comparison to the wild-type parent. In particular, the present invention provides means, including methods and compositions suitable for reducing the immunogenicity of amylase.

### EXAMPLE 5

### Stability of Amylase Variants in Bodywash Solutions and Other Personal Care Products

The stability of various amylase variants is measured using the following protocol.

### Method to Measure Solution Stability

In these experiments, amylase and mutant variants are tested in at least two studies, with the first study involving testing for 30 minutes at 45° C, and the second involving testing for 30 minutes at 50° C. For these tests, 50/50 (w/w) bodywash solutions are prepared by mixing a commercially available bodywash (*e.g*., the bodywash sold under the trademark ZEST ®, from Procter & Gamble), with deionized water. The pH of the buffer blend is approximately 6.8.

The enzymes to be tested are diluted such that their final enzyme concentration in a 50 w/w % BodyWash: deionized water solution produces a change in OD₄₀₅ of 0.5 to 1.0 when 10µl of the enzyme/body wash solution is assayed using SAAPFpNA assay endpoint method. Once the amount of dilution is ascertained, 200 µl of the diluted mixture is placed into 96 well microtiter plate wells. The plate are sealed and placed in a water bath at 40° C, for one study, and at 50° C, for the second study. The plates are removed from the water bath after the desired length of time (*e.g*., 30 or 45 minutes) and 10 µl samples assayed by the endpoint method. The percent of activity remaining is calculated as 100 times the final activity divided by the initial activity.

In some experiments, the variants including the specific residues determined by the I-MUNE® assay system show an increased amount of enzymatic activity remaining and thus have a broader thermal stability than the controls. For example, at 50° C, some variant compounds have a greater percentage activity remaining whereas the control mutant enzyme and/or the wild-type enzyme without the stabilizing residue variants have a lower percentage of activity remaining. In some experiments, all enzymes have enhanced stability in the presence of bodywash at 50°, but control mutant enzyme-[epitopic variants] with different stability variants have even better stability.

Indeed, there are numerous applications in which the variant amylases of the present invention that have reduced immunogenicity find use. In addition to detergents and other cleaning preparations, the variant amylases having reduced immunogenicity also find use in personal care products. The following tables provide the compositions of various products suitable for use in testing. In these tables, the term "minors" encompasses pH modifiers, preservatives, viscosity modifiers, and perfumes. In these tables, the amounts represent approximate weight percent (as provided by the manufacturer), unless otherwise indicated, and are not intended to indicate significant digits.

| MOISTURISING BODYWASH | pH = 7 |
|---|---|
| RAW MATERIAL | Amount |
| Deionized Water | QS |
| Glycerin | 4.0 |
| PEG-6 Caprylic/Capric Glycerides | 4.0 |
| Palm Kernal Fatty acids | 3.0 |
| Sodium Laureth-3 Sulphate | 45.0 |
| Cocamide MEA | 3.0 |
| Sodium Lauroamphoacetate | 25.0 |
| Soybean Oil | 10.0 |
| Polyquaternium-10 (JR30M) | 0.70 |
| Amylase | ~1000ppm |
| Protease | ~1000ppm |

| BODYWASH | pH 6.5 | pH 7 | pH 8.5 |
|---|---|---|---|
| RAW MATERIAL | Amount | Amount | Amount |
| Deionized water | QS | QS | QS |
| Sodium Laureth Sulphate | 12 | 15 | 8 |
| Cocamidopropyl Betaine | 8 | 10 | 15 |
| APG Glucoside (Plantacare 2000¹) | 0 | 2 | 1 |
| Polyquaternium-10 (JR30M) | 0.25 | 0 | 0 |
| Polyquaternium-7 (Mackam 55) | 0 | 0 | 0.7 |
| Protease | ~250ppm | ~500ppm | ~1000ppm |
| Amylase | ~250ppm | ~500ppm | ~1000ppm |

| | | | |
|---|---|---|---|
| 1 - Cognis | | | |

| BODY LOTION | pH 7 | pH 7 | pH 7.5 | pH 7 |
|---|---|---|---|---|
| RAW MATERIAL | Amount | Amount | Amount | Amount |
| DEIONISED WATER | QS | QS | QS | QS |
| GLYCERINE | 8 | 8 | 10 | 12 |
| ISOHEXADECANE | 3 | 3 | 3 | 6 |
| NIACINAMIDE | 0 | 3 | 5 | 6 |
| ISOPROPYL ISOSTEARATE | 3 | 3 | 3 | 3 |
| Polyacrylamide, Isoparaffin, Laureth-7 (Sepigel 305²) | 3 | 3 | 3 | 3 |
| PETROLATUM | 4 | 4 | 4 | 2 |
| NYLON 12 | 2 | 2 | 2.5 | 2.5 |
| DIMETHICONE (DC1403⁴) | 2 | 2 | 2.5 | 2.5 |
| SUCROSE POLYCOTTONSEED OIL | 1.5 | 1.5 | 1.5 | 1.5 |
| Stearyl Alcohol 97% | 1 | 1 | 1 | 1 |
| D PANTHENOL | 1 | 1 | 1 | 1 |
| DL-alphaTOCOPHEROL ACETATE | 1 | 1 | 1 | 1 |
| Cetyl Alcohol 95% | 0.5 | 0.5 | 0.5 | 1 |
| BEHYNYL ALCOHOL | 1 | 1 | 1 | 0.5 |
| EMULGADE PL 68/50 | 0.4 | 0.4 | 0.5 | 0.5 |
| STEARIC ACID | 0.15 | 0.15 | 0.15 | 0.15 |
| Peg-100-stearate (MYRJ 59¹) | 0.15 | 0.15 | 0.15 | 0.15 |
| Amylase | ~50ppm | ~50ppm | ~250ppm | ~1000ppm |
| Protease | ~50ppm | ~50ppm | ~250ppm | ~1000ppm |

| | | | | |
|---|---|---|---|---|
| 1 - Uniqema 2 - Seppic 4 - Dow Corning | | | | |

| ULTRA-HIGH MOISTURISING FACIAL CREAM/LOTION | pH 7 | pH 7 |
|---|---|---|
| RAW MATERIAL | Amount | Amount |
| Deionized water | QS | QS |
| Glycerin | 12 | 5 |
| PEG 400⁶ | 0 | 10 |
| Niacinamide | 5 | 7 |
| Isohexadecane | 5 | 5 |
| Dimethicone (DC1403³) | 3 | 2 |
| Polyacrylamide, Isoparaffin, Laureth-7 (Sepigel 305¹) | 3 | 3 |
| Isopropyl Isostearate | 2 | 2 |
| Polymethylsilsesquioxane | 2 | 2 |
| Cetyl Alcohol 95% | 1 | 1 |
| Sucrose polycottonseed oil | 1 | 1 |
| D-Panthenol | 1 | 1 |
| Vitamin E (Tocopherol Acetate) | 1 | 1 |
| Stearyl Alcohol 95% | 0.5 | 0.5 |
| Cetearyl Glucoside | 0.5 | 0.5 |
| Titanium dioxide | 0.3 | 0.3 |
| Stearic Acid | 0.15 | 0.15 |
| PEG-100-Stearate (Myrj 59⁴) | 0.15 | 0.15 |
| Amylase | ~500ppm | ~500ppm |
| Protease | ~500ppm | ~500ppm |

| | | |
|---|---|---|
| 1 - Seppic 3 - Dow Corning 4 - Uniqema 5 - Scher Chemicals 6 - Dow Chemicals | | |

| FACIAL MOISTURIZING CREAM | pH 7 | pH 7 | pH 7.5 |
|---|---|---|---|
| RAW MATERIAL | Amount | Amount | Amount |
| Deionized water | QS | QS | QS |
| Glycerin | 3 | 5 | 10 |
| Petrolatum | 3 | 3 | 0 |
| Cetyl Alcohol 95% | 1.5 | 1.5 | 1 |
| Dimethicone Copolyol (DC 3225C ⁴) | 2 | 2 | 2 |
| Isopropyl Palmitate | 1 | 1 | 0.5 |
| Carbomer 954² | 0.7 | 0.7 | 0.7 |
| Dimethicone (DC 200/350cs⁴) | 1 | 1 | 1 |
| Stearyl Alcohol 97% | 0.5 | 0.5 | 1 |
| Stearic acid | 0.1 | 0.1 | 0.1 |
| Peg-100-stearate (MYRJ 59¹) | 0.1 | 0.1 | 0.1 |
| Titanium Dioxide | 0.3 | 0.3 | 0.3 |
| Amylase | ~50ppm | ~250ppm | ~1000ppm |
| Protease | ~50ppm | ~250ppm | ~1000ppm |

| | | | |
|---|---|---|---|
| 1 - Uniqema 2 - BF Goodrich 4 - Dow Corning | | | |

### EXAMPLE 6

### Cleaning Compositions

In addition to the compositions described above, means to develop cleaning compositions having particular characteristics are provided herein. Indeed, various cleaning compositions that comprise modified amylases are provided. An effective amount of one or more amylase enzymes described above are preferably included in compositions useful for cleaning a variety of surfaces in need of proteinaceous stain removal. Such cleaning compositions include detergent compositions for cleaning hard surfaces; detergent compositions for cleaning fabrics; dishwashing compositions; oral cleaning compositions; and denture cleaning compositions. It is intended that these compositions be provided in any form suitable for the particular intended use. Preferably, the cleaning compositions comprise from about 0.0001% to about 10% of one or more amylase enzymes, more preferably from about 0.001 % to about 1 %, and more preferably still from about 0.001% to about 0.1 %. Several examples of various cleaning compositions wherein the amylase enzymes find use are discussed in further detail below. All parts, percentages and ratios used herein are by weight unless otherwise specified.

### A. Cleaning Compositions for Hard Surfaces, Dishes, and Fabrics

The amylase enzymes (*e.g*., the variant amylases) of the described herein find use in any detergent composition where high sudsing and/or good insoluble substrate removal are desired. Thus, the amylase enzymes find use with various conventional ingredients to provide fully-formulated hard-surface cleaners, dishwashing compositions, fabric laundering compositions and the like. These compositions are suitable for use in any form (*e.g*., liquid, granules, bars, etc.) acceptable for the particular application. In addition, these compositions are also suitable for use in commercially available "concentrated" detergents which contain as much as 30%-60% by weight of surfactants.

The cleaning compositions may contain various anionic, nonionic, zwitterionic, etc., surfactants. Such surfactants are typically present at levels of from about 0.1 % to about 60%, preferably from about 1 % to about 35%, of the compositions. Suitable surfactants include, but are not limited to the conventional C₁₁-C₁₈ alkyl benzene sulfonates and primary and random alkyl sulfates, the C₁₀-C₁₈ secondary (2,3) alkyl sulfates of the formulas CH₃ (CH₂)x(CHOSO₃).sup.- M.sup.+)CH₃, and CH₃ (CH₂)_{y}(CHOSO₃.sup.- M.sup.+) CH₂ CH₃, wherein x and (y+1) are integers of at least about 7, preferably at least about 9, and M is a water-solubilizing cation, especially sodium, the C₁₀-C₁₈ alkyl alkoxy sulfates (especially EO 1-7 ethoxy sulfates), C₁₀-C₁₈ alkyl alkoxy carboxylates (especially the EO 1-7 ethoxycarboxylates), the C₁₀-C₁₈ alkyl polyglycosides, and their corresponding sulfated polyglycosides, C₁₂-C₁₈ alpha-sulfonated fatty acid esters, C₁₂-C₁₈ alkyl and alkyl phenol alkoxylates (especially ethoxylates and mixed ethoxy/propoxy), C₁₂-C₁₈ betaines and sulfobetaines ("sultaines"), C₁₀-C₁₈ amine oxides, C₈-C₂₄ sarcosinates (especially oleoyl sarcosinate), and the like. The alkyl alkoxy sulfates (AES) and alkyl alkoxy carboxylates (AEC) are preferred herein. Furthermore, use of such surfactants in combination with the aforesaid amine oxide and/or betaine or sultaine surfactants is also preferred, depending on the desires of the formulator. Other conventional useful surfactants are known to those in the art, including, but not limited to the particularly useful surfactants such as the C₁₀-C₁₈ N-methyl glucamides (*See*, US Pat. No. 5, 194,639).

The compositions may comprise member(s) of the class of nonionic surfactants which are condensates of ethylene oxide with a hydrophobic moiety to provide a surfactant having an average hydrophilic-lipophilic balance (HLB) in the range from 5 to 17, preferably from 6 to 14, more preferably from 7 to 12. The hydrophobic (lipophilic) moiety may be aliphatic or aromatic in nature and the length of the polyoxyethylene group which is condensed with any particular hydrophobic group can be readily adjusted to yield a water-soluble compound having the desired degree of balance between hydrophilic and hydrophobic elements. Especially preferred are the C₉-C₁₅ primary alcohol ethoxylates (or mixed ethoxy/propoxy) containing 3-8 moles of ethylene oxide per mole of alcohol, particularly the C₁₄-C₁₅ primary alcohols containing 6-8 moles of ethylene oxide per mole of alcohol, the C₁₂-C₁₅ primary alcohols containing 35 moles of ethylene oxide per mole of alcohol, and mixtures thereof.

A wide variety of other ingredients useful in detergent cleaning compositions find use in the compositions herein, including other active ingredients, carriers, hydrotropes, processing aids, dyes or pigments, solvents for liquid formulations, etc. For an additional increment of sudsing, suds boosters such as the C₁₀-C₁₆ alkolamides can be incorporated into the compositions, typically at about 1% to about 10% levels. The C₁₀-C₁₄ monoethanol and diethanol amides illustrate a typical class of such suds boosters. Use of such suds boosters with high sudsing adjunct surfactants such as the amine oxides, betaines and sultaines noted above is also advantageous. If desired, soluble magnesium salts such as MgCl₂, MgSO₄, and the like, can be added at levels of, typically, from about 0.1% to about 2%, to provide additional sudsing.

The liquid detergent compositions herein typically contain water and other solvents as carriers. Low molecular weight primary or secondary alcohols (*e.g*., methanol, ethanol, propanol, and isopropanol) are suitable. Monohydric alcohols are preferred for solubilizing surfactants, but polyols such as those containing from about 2 to about 6 carbon atoms and from about 2 to about 6 hydroxy groups (*e.g*., 1,3-propanediol, ethylene glycol, glycerine, and 1,2-propanediol) also find use in the detergents of the present invention. The compositions may contain about 90%, or from about 10% to about 50% of such carriers.

The detergent compositions herein are preferably formulated such that during use in aqueous cleaning operations, the wash water has a pH between about 6.8 and about 11.0. Thus, finished products are typically formulated at this range. Techniques for controlling pH at recommended usage levels include the use of buffers, alkalis, acids, etc., and are well known to those skilled in the art.

When formulating the hard surface cleaning compositions and fabric cleaning compositions of the present invention, the formulator may wish to employ various builders at levels from about 5% to about 50% by weight. Typical builders include the 1-10 micron zeolites, polycarboxylates such as citrate and oxydisuccinates, layered silicates, phosphates, and the like. Other conventional builders are known to those in the art and are suitable for inclusion in the compositions of the present invention.

Likewise, the formulator may wish to employ various additional enzymes, such as cellulases, lipases, amylases, peroxidases, and proteases in such compositions, typically at levels of from about 0.001 % to about 1 % by weight. Various detersive and fabric care enzymes are well-known in the laundry detergent art and are suitable for inclusion in the compositions.

Various bleaching compounds, such as the percarbonates, perborates and the like, also find use in the compositions of the present invention. These bleaching compounds are typically present at levels from about 1% to about 15% by weight. If desired, such compositions can also contain bleach activators such as tetraacetyl ethylenediamine, nonanoyloxybenzene sulfonate, and the like, which are also known in the art. Usage levels of such compounds typically range from about 1% to about 10% by weight.

Various soil release agents, especially of the anionic oligoester type, various chelating agents, especially the aminophosphonates and ethylenediaminedisuccinates, various clay soil removal agents, especially ethoxylated tetraethylene pentamine, various dispersing agents, especially polyacrylates and polyasparatates, various brighteners, especially anionic brighteners, various dye transfer inhibiting agents, such as polyvinyl pyrrolidone, various suds suppressors, especially silicones and secondary alcohols, various fabric softeners, especially smectite clays and clay floculating polymers (*e.g*., poly(oxy ethylene)), and the like all find use in the compositions of the present invention, most typically at levels ranging from about 1% to about 35% by weight.

Enzyme stabilizers also find use in the cleaning compositions of the present invention. Such enzyme stabilizers include, but are not limited to propylene glycol (preferably from about 1% to about 10%), sodium formate (preferably from about 0.1 % to about 1 %) and calcium formate (preferably from about 0.1% to about 1%).

### 1. Hard Surface Cleaning Compositions

Hard surface cleaning compositions preferably comprise an effective amount of one or more amylase enzymes (*e.g*., variant amylases), preferably from about 0.0001 % to about 10%, more preferably from about 0.001 % to about 5%, more preferably still from about 0.001 % to about 1% by weight of active amylase enzyme of the composition. In addition to comprising one or more amylase enzymes, such hard surface cleaning compositions typically comprise at least one amylase, at least one protease, a surfactant, and a water-soluble sequestering builder. However, in certain specialized products such as spray window cleaners, the surfactants are sometimes not used since they may produce a filmy/streaky residue on the glass surface.

The surfactant component, when present, may comprise as little as 0.1% of the compositions herein, but typically the compositions will contain from about 0.25% to about 10%, more preferably from about 1% to about 5% of surfactant.

Typically the compositions will contain from about 0.5% to about 50% of a detergency builder, preferably from about 1% to about 10%. Preferably, the pH should be in the range of about 8 to 12. Conventional pH adjustment agents such as sodium hydroxide, sodium carbonate or hydrochloric acid can be used if adjustment is necessary.

Optionally, at least one solvent is included in the compositions. Useful solvents include, but are not limited to, glycol ethers such as diethyleneglycol monohexyl ether, diethyleneglycol monobutyl ether, ethyleneglycol monobutyl ether, ethyleneglycol monohexyl ether, propyleneglycol monobutyl ether, dipropyleneglycol monobutyl ether, and diols such as 2,2,4-trimethyl-1,3pentanediol and 2-ethyl-1,3-hexanediol. When used, such solvents are typically present at levels of from about 0.5% to about 15%, preferably from about 3% to about 11%.

Additionally, highly volatile solvents such as isopropanol or ethanol find use in the present compositions, in order to facilitate faster evaporation of the composition from surfaces when the surface is not rinsed after "full strength" application of the composition to the surface. When used, volatile solvents are typically present at levels of from about 2% to about 12% in the compositions.

The hard surface cleaning composition is illustrated by the following examples. In the following examples, reference to "Amylase#" in the examples is to the variant useful in compositions having a reduced immunogenic responding amylase variant of percentages of 0.10, 0.20, 0.10, 0.05, 0.03, and 0.02.

| **Liquid Hard Surface Cleaning Compositions** | | | | | | |
|---|---|---|---|---|---|---|
| **Component** | **Example No.** | | | | | |
| | **1** | **2** | **3** | **4** | **5** | **6** |
| EDTA** | | | 2.90 | 2.90 | | |
| Na Citrate | | | | | 2.90 | 2.90 |
| NaC₁₂ Alkylbenzene | 1.95 | | 1.95 | | 1.95 | |
| NaC₁₂ Alkylsulfate | | 2.20 | | 2.20 | | 2.20 |
| NaC₁₂ (ethoxy)*** | | 2.20 | | 2.20 | | 2.20 |
| C₁₂ Dimethylamine | | 0.50 | | 0.50 | | 0.50 |
| Na Cumene sulfonate | 1.30 | | 1.30 | | 1.30 | |
| Hexyl Carbitol*** | 6.30 | 6.30 | 6.30 | 6.30 | 6.30 | 6.30 |
| Water**** | Balance to 100% | | | | | |

| | | | | | | |
|---|---|---|---|---|---|---|
| ** Na₄ Ethylenediamine diacetic acid *** Diethyleneglycol monohexyl ether **** All formulae adjusted to pH 7. | | | | | | |

In the above examples, additional amylases (*e.g.*, variant amylases) may be substituted with substantially similar results. In addition, in of the above examples, any combination of the reduced immunogenic amylases (*e.g.,* variant amylases) may be substituted in the above formulations with substantially similar results.

The following Table provides sample compositions suitable for cleaning hard surfaces and removing mildew. The product compositions are typically at approximately pH 7.

| **Spray Compositions for Cleaning Hard Surfaces and Removing Household Mildew** | | | | | | |
|---|---|---|---|---|---|---|
| **Component** | **Example No.** | | | | | |
| | **7** | **8** | **9** | **10** | **11** | **12** |
| Amylase # | 0.20 | 0.05 | 0.10 | 0.30 | 0.20 | 0.30 |
| Amylase #+14 | | | | | 0.30 | 0.10 |
| Protease # | 0.20 | 0.05 | 0.10 | 0.30 | 0.20 | 0.30 |
| Protease #+14 | | | | | 0.30 | 0.10 |
| Sodium octyl sulfate | 2.00 | 2.00 | 2.00 | 2.00 | 2.00 | 2.00 |
| Sodium dodecyl sulfate | 4.00 | 4.00 | 4.00 | 4.00 | 4.00 | 4.00 |
| NaOH | 0.80 | 0.80 | 0.80 | 0.80 | 0.80 | 0.80 |
| Silicate (Na) | 0.04 | 0.04 | 0.04 | 0.04 | 0.04 | 0.04 |
| Perfume | 0.35 | 0.35 | 0.35 | 0.35 | 0.35 | 0.35 |
| Water | Balance to 100% | | | | | |

In these Examples, any combination of the amylase enzymes (*e.g*., variant amylases) is substituted in with substantially similar results.

### 2. Dishwashing Compositions

Dishwashing compositions comprising one or more amylase enzymes (*e.g*., mutant amylases) are also provided. Preferred dishwashing compositions are illustrated below. Amylases are included with percentages at 0.5, 0.4, 0.1, 0.05, 0.03, and 0.02. In these compositions, the product pH is adjusted to 7.

| **Dishwashing Compositions** | | | | | | |
|---|---|---|---|---|---|---|
| **Component** | **Example No.** | | | | | |
| | **13** | **14** | **15** | **16** | **17** | **18** |
| C₁₂ -C₁₄ N-methyl- | 0.90 | 0.90 | 0.90 | 0.90 | 0.90 | 0.90 |
| C₁₂ ethoxy (1) sulfate | 12.0 | 12.0 | 12.0 | 12.0 | 12.0 | 12.0 |
| 2-m ethyl undecanoic acid | 4.50 | 4.50 | | 4.50 | 4.50 | |
| C₁₂ ethoxy (2) carboxylate | 4.50 | 4.50 | 4.50 | 4.50 | 4.50 | 4.50 |
| C₁₂ alcohol ethoxylate (4) | 3.00 | 3.00 | 3.00 | 3.00 | 3.00 | 3.00 |
| C₁₂ amine oxide | 3.00 | 3.00 | 3.00 | 3.00 | 3.00 | 3.00 |
| Sodium cumene sulfonate | 2.00 | 2.00 | 2.00 | 2.00 | 2.00 | 2.00 |
| Ethanol | 4.00 | 4.00 | 4.00 | 4.00 | 4.00 | 4.00 |
| Mg Supp⁺⁺ (MgCl₂) | 0.20 | 0.20 | 0.20 | 0.20 | 0.20 | 0.20 |
| Ca Supp⁺⁺ (CaCl₂) | 0.40 | 0.40 | 0.40 | 0.40 | 0.40 | 0.40 |
| Water | Balance to 100% | | | | | |

In the immediately above examples the amylases may be (*e.g*., variant amylases) may be substituted in the above formulations, with substantially similar results. Furthermore, the immediately above examples, any combination of the amylase enzymes (*e.g*., variant amylases), among others, is substituted in the above formulations with substantially similar results.

| **Granular Automatic Dishwashing Compositions** | | | |
|---|---|---|---|
| **Component** | **Example** | | |
| | **A** | **B** | **C** |
| Citric acid | 15.0 | | |
| Citrate | 4.0 | 29.0 | 15.0 |
| Acrylate/methacrylate copolymer | 6.0 | | 6.0 |
| Acrylic acid maleic acid copolymer | | 3.7 | |
| Dry add carbonate | 9.0 | | 20.0 |
| Alkali metal silicate | 8.5 | 17.0 | 9.0 |
| Paraffin | | 0.5 | |
| Benzotriazole | | 0.3 | |
| Termamyl 60T | 1.5 | 1.5 | 1.0 |
| Amylase #4 | 1.6 | 1.6 | 1.6 |
| Protease #4 (4.6% pril) | 1.6 | 1.6 | 1.6 |
| Percarbonate (AvO) | 1.5 | | |
| Perborate monohydrate | | 0.3 | 1.5 |
| Perborate tetrahydrate | | 0.9 | |
| Tetraacetylethylene diamine | 3.8 | 4.4 | |
| Diethylene triamine penta methyphosphonic acid (Mg salt) | 0.13 | 0.13 | 0.13 |
| Alkyl ethoxy sulphate-3x ethoxylated | 3.0 | | |
| Alkyl ethoxy propoxy nonionic surfactant | | | |
| Suds suppressor | 2.0 | | |
| Olin SLF18 nonionic surfactant | | | |
| Sulfate | | | |

In the immediately above formulations a reduced immunogenic amylase (*e.g*., a variant amylase) useful in the present invention is substituted therein with substantially similar results. Also in the immediately above formulations, any combination of the amylases useful in the present invention (*e.g*., variant amylases) recited herein can be substituted in with substantially similar results.

### 3. Fabric Cleaning Compositions

Also provided are fabric cleaning compositions comprising one or more amylase enzymes (*e.g*., variant amylases).

### a. Granular Fabric Cleaning

The granular fabric cleaning compositions contain an effective amount of one or more amylase enzymes (*e.g*., variant amylases), preferably from about 0.001% to about 10%, more preferably from about 0.005% to about 5%, more preferably from about 0.01 % to about 1% by weight of active amylase enzyme of the composition. In addition to one or more amylase enzymes, the granular fabric cleaning compositions typically comprise at least one surfactant, one or more builders, and, in some cases, a bleaching agent. Granular fabric cleaning composition embodiments of the present invention are illustrated by the following examples.

| **Granular Fabric Cleaning Compositions** | | | | |
|---|---|---|---|---|
| **Component** | **Example No.** | | | |
| | **20** | **21** | **22** | **23** |
| Amylase | 0.10 | 0.20 | 0.03 | 0.05 |
| Amylase | | | 0.02 | 0.05 |
| Protelase (4% Prill) | 0.10 | 0.20 | 0.03 | 0.05 |
| Protease (4% Prill) | | | 0.02 | 0.05 |
| C₁₃ linear alkyl benzene sulfonate | 22.0 | 22.0 | 22.0 | 22.0 |
| Phosphate (as sodium tripoly-phosphates) | 23.0 | 23.0 | 23.0 | 23.0 |
| Sodium carbonate | 23.0 | 23.0 | 23.0 | 23.0 |
| Sodium silicate | 12.0 | 14.0 | 14.0 | 14.0 |
| Zeolite | 8.20 | 8.20 | 8.20 | 8.20 |
| Chelant (diethylaenetriamine-pentaacetic acid) | 0.40 | 0.40 | 0.40 | 0.40 |
| Sodium sulfate | 5.50 | 5.50 | 5.50 | 5.50 |
| Water | Balance to 100% | | | |

In the immediately above formulations a reduced immunogenic amylase useful in the present invention (*e.g*., variant amylases) is substituted therein with substantially similar results. Also in the immediately above formulations, any combination of the amylases (*e.g*., variant amylases) useful in the present invention recited herein can be substituted in with substantially similar results.

| **Granular Fabric Cleaning Composition** | | | | |
|---|---|---|---|---|
| **Component** | **Example No.** | | | |
| | **24** | **25** | **26** | **27** |
| Amylase # | 0.10 | 0.20 | 0.03 | 0.05 |
| Amylase # +1 | | | 0.02 | 0.05 |
| Protease # (4% Prill) | 0.10 | 0.20 | 0.03 | 0.05 |
| Protease # +1 (4% Prill) | | | 0.02 | 0.05 |
| C₁₂ alkyl benzene sulfonate | 12.0 | 12.0 | 12.0 | 12.0 |
| Zeolite A (1-10 µm) | 26.0 | 26.0 | 26.0 | 26.0 |
| 2-butyl octanoic acid | 4.0 | 4.0 | 4.0 | 4.0 |
| C₁₂-C₁₄ secondary (2,3) | 5.0 | 5.0 | 5.0 | 5.0 |
| Sodium citrate | 5.0 | 5.0 | 5.0 | 5.0 |
| Optical brightener | 0.10 | 0.10 | 0.10 | 0.10 |
| Sodium sulfate | 17.0 | 17.0 | 17.0 | 17.0 |
| Fillers, water, minors | Balance to 100% | | | |

In the immediately above formulations a reduced immunogenic amylase (*e.g*., variant amylase) useful in the present invention is substituted therein with substantially similar results. Also in the immediately above formulations, any combination of the amylases useful in the present invention (*e.g*., variant amylases) can be substituted in with substantially similar results.

| **Granular Fabric Cleaning Compositions** | | |
|---|---|---|
| **Component** | **Example No.** | |
| | **28** | **29** |
| Linear alkyl benzene sulphonate | 11.4 | 10.7 |
| Tallow alkyl sulphate | 1.8 | 2.4 |
| C₁₄₋₁₅ alkyl sulphate | 3.0 | 3.10 |
| C₁₄₋₁₅ alcohol 7 times ethoxylated | 4.0 | 4.0 |
| Tallow alcohol 11 times ethoxylated | 1.8 | 1.8 |
| Dispersant | 0.07 | 0.1 |
| Silicone fluid | 0.80 | 0.80 |
| Trisodium citrate | 14.0 | 15.0 |
| Citric acid | 3.0 | 2.5 |
| Zeolite | 32.5 | 32.1 |
| Maleic acid acrylic acid copolymer | 5.0 | 5.9 |
| Diethylene triamine penta methylene | 1.0 | 0.20 |
| Protease # (4% Prill) | 0.30 | 0.30 |
| Lipase | 0.36 | 0.40 |
| Amylase | 0.30 | 0.30 |
| Sodium silicate | 2.0 | 2.5 |
| Sodium sulphate | 3.5 | 5.2 |
| Polyvinyl pyrrolidone | 0.3 | 0.5 |
| Perborate | 0.5 | 1 |
| Phenol sulphonate | 0.1 | 0.2 |
| Peroxidase | 0.1 | 0.1 |
| Minors | Up to 100 | |

**Granular Fabric Cleaning Compositions**

| **Component** | **Example No.** | |
|---|---|---|
| | **30** | **31** |
| Sodium linear C₁₂ alkyl benzene sulphonate | 6.5 | 8.0 |
| Sodium sulphate | 15.0 | 18.0 |
| Zeolite | 26.0 | 22.0 |
| Sodium nitrilotriacetate | 5.0 | 5.0 |
| Polyvinyl pyrrolidone | 0.5 | 0.7 |
| Tetraacetylethylene diamine | 3.0 | 3.0 |
| Boric acid | 4.0 | |
| Perborate | 0.5 | 1 |
| Phenol sulphonate | 0.1 | 0.2 |
| Protease #4 (4% Prill) | 0.4 | 0.4 |
| Amylase #4 | 0.4 | 0.4 |
| Fillers (e.g., silicates, carbonates, perfumes) | Up to 100 | |

Additionally, compact granular fabric cleaning compositions such as the following are provided. The ingredients are provided in terms of the weight percent. Composition 1: alkyl sulphate (8.0), alkyl ethoxy sulphate (2.0), a mixture of C25 and C45 alcohol 3 and 7 times ethoxylated (6.0), polyhydroxy fatty acid amide (2.5), Zeolite (17.0), layered silicate/citrate (16.0), carbonate (7.0), maleic acid acrylic acid copolymer (5.0), soil release polymer (0.4), carboxymethyl cellulose (0.4), poly(4-vinylpyridine)-N-oxide (0.1), copolymer of vinylimidazole and vinylpyrrolidone (0.1), PEG-2000 (0.2), amylase # (0.5), protease (4% Prill) (0.5), lipase (0.2), cellulase (0.2), tetracetylethylene diamine (6.0), percarbonate (22.0), ethylene diamine disuccinic acid (0.3), suds suppressor (3.5), disodium-4,4'-bis(2-morpholino-4-anilino-s-triazin-6-ylamino)stilbene-2,2'-disulphonate (0.25), Disodium-4,4'-bis(2-sulfostyril)biphenyl (0.05), and a combination of water, perfume and minors (up to 100).

In an alternative granular fabric cleaning composition, the following ingredients are provided. The ingredients are provided in terms of the weight percent. Composition 2: linear alkyl benzene sulphonate (7.6), C₁₆-C₁₈ alkyl sulfate (1.3), C₁₄₋₁₅ alcohol 7 times ethoxylated (4.0), coco-alkyl-dimethyl hydroxyethyl ammonium chloride (1.4), dispersant (0.07), silicone fluid (0.8), trisodium citrate (5.0), Zeolite 4A (15.0), maleic acid acrylic acid copolymer (4.0), diethylene triamine penta methylene phosphonic acid (0.4), perborate (15.0), tetraacetylethylene diamine (5.0), smectite clay (10.0), poly (oxy ethylene) (MW 300,000) (0.3), amylase #, amylase # (0.4), protease (4% Prill) (0.4), lipase (0.2), amylase (0.3), cellulase (0.2), sodium silicate (3.0), sodium carbonate (10.0), carboxymethyl cellulose (0.2), brighteners (0.2), and a mixture of water, perfume and minors (up to 100).

In yet another alternative granular fabric cleaning composition, the following ingredients are provided. The ingredients are provided in terms of the weight percent. Composition 2: linear alkyl benzene (6.92), tallow alkyl sulfate (2.05), C₁₄₋₁₅ alcohol 7 times ethoxylated (4.4), C₁₂₋₁₅ alkyl ethoxy sulfate - 3 times ethoxylated (0.16), Zeolite (20.2), citrate (5.5), carbonate (15.4), silicate (3.0), maleic acid acrylic acid copolymer (4.0), carboxymethyl cellulase (0.31), soil release polymer (0.30), amylase #, protease # (4% Prill) (0.2), lipase (0.36), cellulase (0.13), perborate tetrahydrate (11.64), perborate monohydrate (8.7), tetraacetylethylene diamine (5.0), diethylene tramine penta methyl phosphonic acid (0.38), magnesium sulfate (0.40), brightener (0.19), a mixture of perfume, silicone, and suds suppressors (0.85), and minors (up to 100).

In the immediately above formulations a reduced immunogenic amylase (*e.g*., a variant amylase) is substituted therein with substantially similar results. Also in the immediately above formulations, any combination of the amylases useful in the present invention (*e.g*., variant amylases) can be substituted in with substantially similar results.

### b. Liquid Fabric Cleaning Compositions

Liquid fabric cleaning compositions provided herein comprise an effective amount of one or more amylase enzymes (*e.g*., variant amylases), preferably from about 0.0001 % to about 10%, more preferably from about 0.001 % to about 1%, and most preferably from about 0.001 % to about 0.1 %, by weight of active amylase enzyme of the composition. Such liquid fabric cleaning compositions typically additionally comprise an anionic surfactant, a fatty acid, a water-soluble detergency builder and water. The liquid fabric cleaning composition is illustrated by the following examples.

| **Liquid Fabric Cleaning Compositions** | | | | | |
|---|---|---|---|---|---|
| **Component** | **Example No**. | | | | |
| | **35** | **36** | **37** | **38** | **39** |
| Amylase # | 0.05 | 0.03 | 0.30 | 0.03 | 0.10 |
| Amylase # +1 | | | | 0.01 | 0.20 |
| Protease # | 0.05 | 0.03 | 0.30 | 0.03 | 0.10 |
| Protease # +1 | | | | 0.01 | 0.20 |
| C₁₂-C₁₄ alkyl sulfate, Na | 20.0 | 20.0 | 20.0 | 20.0 | 20.0 |
| 2-butyl octanoic acid | 5.0 | 5.0 | 5.0 | 5.0 | 5.0 |
| Sodium citrate | 1.0 | 1.0 | 1.0 | 1.0 | 1.0 |
| C₁₀ alcohol ethoxylate (3) | 13.0 | 13.0 | 13.0 | 13.0 | 13.0 |
| Monethanolamine | 2.5 | 2.5 | 2.5 | 2.5 | 2.5 |
| Water/propylene glycol/ethanol | Balance to 100% (100:1:1) | | | | |

In the immediately above formulations a reduced immunogenic amylase (*e.g*., a variant amylase) is substituted therein with substantially similar results. Also in the immediately above formulations, any combination of the amylases useful in the present invention (*e.g*., variant amylases) can be substituted in with substantially similar results.

| **Liquid Fabric Cleaning Compositions** | | |
|---|---|---|
| **Component** | **Example No.** | |
| | **40** | **41** |
| C₁₂₋₁₄ alkyl succinic acid | 3.0 | 8.0 |
| Citric acid monohydrate | 10.0 | 15.0 |
| Sodium C₁₂₋₁₅ alkyl sulphate | 8.0 | 8.0 |
| Sodium sulfate of C₁₂₋₁₅ alcohol 2 times ethoxylated | | 3.0 |
| C₁₂₋₁₅ alcohol 7 times ethoxylated | | 8.0 |
| C₁₂₋₁₅ alcohol 5 times ethoxylated | 8.0 | |
| Diethylene triamine penta (methylene phosphonic acid) | 0.2 | |
| Oleic acid | 1.8 | |
| Ethanol | 4.0 | 4.0 |
| Propanediol | 2.0 | 2.0 |
| Amylase # | 0.2 | 0.2 |
| Polyvinyl pyrrolidone | 1.0 | 2.0 |
| Suds suppressor | 0.15 | 0.15 |
| NaOH | up to pH 7.5 | |
| Perborate | 0.5 | 1.0 |
| Phenol sulphonate | 0.1 | 0.2 |
| Peroxidase | 0.4 | 0.1 |
| Water and minors | Up to 100 | |

In the immediately above formulations a reduced immunogenic amylase (*e.g*., a variant amylase) is substituted therein with substantially similar results. Also in the immediately above formulations, any combination of the amylases useful in the present invention (*e.g*., variant amylases) can be substituted in with substantially similar results.

### c. Bar Fabric Cleaning Compositions

Bar fabric cleaning compositions of the present invention suitable for hand-washing soiled fabrics contain an effective amount of one or more amylase enzymes (*e.g*., variant amylases), preferably from about 0.001 % to about 10%, more preferably from about 0.01 % to about 1% by weight of the composition. The bar fabric cleaning composition embodiments of the present invention is illustrated by the following examples.

| **Bar Fabric Cleaning Composition** | | | | |
|---|---|---|---|---|
| **Component** | **Example No.** | | | |
| | **42** | **43** | **44** | **45** |
| Protease # | 0.3 | | 0.1 | 0.02 |
| Protease # + 1 | | | 0.4 | 0.03 |
| Amylase # | 0.3 | | 0.1 | 0.02 |
| Amylase # +1 | | | 0.4 | 0.03 |
| C₁₂₋C₁₆ alkyl sulfate, Na | 20.0 | 20.0 | 20.0 | 20.0 |
| C₁₂-Cₗ₄-N-methyl glucamide | 5.0 | 5.0 | 5.0 | 5.0 |
| C₁₁-C₁₃ alkyl benzene sulfonate, Na | 10.0 | 10.0 | 10.0 | 10.0 |
| Sodium carbonate | 25.0 | 25.0 | 25.0 | 25.0 |
| Sodium pyrophosphate | 7.0 | 7.0 | 7.0 | 7.0 |
| Sodium tripolyphosphate | 7.0 | 7.0 | 7.0 | 7.0 |
| Zeolite A (0.1-10 µm) | 5.0 | 5.0 | 5.0 | 5.0 |
| Carboxymethylcellulose | 0.2 | 0.2 | 0.2 | 0.2 |
| Polyacrylate (MW 1400) | 0.2 | 0.2 | 0.2 | 0.2 |
| Coconut monethanolamide | 5.0 | 5.0 | 5.0 | 5.0 |
| Brightener, perfume | 0.2 | 0.2 | 0.2 | 0.2 |
| CaSO₄ | 1.0 | 1.0 | 1.0 | 1.0 |
| MgSO₄ | 1.0 | 1.0 | 1.0 | 1.0 |
| Water | 4.0 | 4.0 | 4.0 | 4.0 |
| Fillers (*e.g.,* CaCO3, talc, clay, silicates, etc.) | Balance to 100% | | | |

In the immediately above formulations a reduced immunogenic amylase (*e.g*., a variant amylase) is substituted therein with substantially similar results. Also in the immediately above formulations, any combination of the amylases useful in the present invention (*e.g*., variant amylases) can be substituted in with substantially similar results.

### B. Additional Cleaning Compositions

In addition to the hard surface cleaning, dishwashing and fabric cleaning compositions discussed above, one or more amylase enzymes (*e.g*., variant amylases) find use as components of various other cleaning compositions where hydrolysis of an insoluble substrate is desired. Such additional cleaning compositions include, but are not limited to oral cleaning compositions, denture cleaning compositions, and contact lens cleaning compositions, as well as other personal care cleaning compositions.

### 1. Oral Cleaning Compositions

Pharmaceutically-acceptable amounts of one or more amylase enzymes (*e.g*., variant amylases) may be included in compositions useful for removing proteinaceous stains from teeth or dentures. Preferably, oral cleaning compositions comprise from about 0.0001% to about 20% of one or more amylase enzymes, more preferably from about 0.001 % to about 10%, more preferably still from about 0.01 % to about 5%, by weight of the composition, and a pharmaceutically-acceptable carrier. Typically, the pharmaceutically-acceptable oral cleaning carrier components of the oral cleaning components of the oral cleaning compositions will generally comprise from about 50% to about 99.99%, preferably from about 65% to about 99.99%, more preferably from about 65% to about 99%, by weight of the composition.

The pharmaceutically-acceptable carrier components and optional components which may be included in the oral cleaning compositions of the present invention are well known to those skilled in the art. A wide variety of composition types, carrier components and optional components useful in the oral cleaning compositions are disclosed in US Pat. No. 5,096,700; US Pat. No. 5,028,414; and US Pat. No. 5,028,415. Oral cleaning composition are illustrated by the following examples.

| **Oral Dentrifice Cleaning Composition** | | | | |
|---|---|---|---|---|
| **Component** | **Example No.** | | | |
| | **46** | **47** | **48** | **49** |
| Amylase # | 2.0 | 3.5 | 1.5 | 2.0 |
| Protease | 2.0 | 3.5 | 1.5 | 2.0 |
| Sorbitol (70% aq. soln.) | 35.0 | 35.0 | 35.0 | 35.0 |
| PEG-6* | 1.0 | 1.0 | 1.0 | 1.0 |
| Silica dental abrasive** | 20.0 | 20.0 | 20.0 | 20.0 |
| Sodium fluoride | 0.243 | 0.243 | 0.243 | 0.243 |
| Titanium oxide | 0.5 | 0.5 | 0.5 | 0.5 |
| Sodium saccharin | 0.286 | 0.286 | 0.286 | 0.286 |
| Sodium alkyl sulfate (27.9%) | 4.0 | 4.0 | 4.0 | 4.0 |
| Flavor | 1.04 | 1.04 | 1.04 | 1.04 |
| Carboxyvinyl polymer*** | 0.30 | 0.30 | 0.30 | 0.30 |
| Carrageenan**** | 0.8 | 0.8 | 0.8 | 0.8 |
| Water | Balance to 100% | | | |

| | | | | |
|---|---|---|---|---|
| *PEG-6-Polyethylene glycol, having MW of 600 **Precipitated silica identified as Zeodent 119 (J.M. Huber). ***Carbopol (B.F. Goodrich Chemical Co.) ****lota carrageenan (Hercules Chemical Co.). | | | | |

In the immediately above formulations a reduced immunogenic amylase (*e.g.,* a variant amylase) is substituted therein with substantially similar results. Also in the immediately above formulations, any combination of the amylases (*e.g*., variant amylases) can be substituted in with substantially similar results.

| **Mouthwash Compositions** | | | | |
|---|---|---|---|---|
| **Component** | **Example No.** | | | |
| | **50** | **51** | **52** | **53** |
| Amylase # | 3.0 | 7.5 | 1.0 | 5.0 |
| Protease # | 3.0 | 7.5 | 1.0 | 5.0 |
| SDA 40 Alcohol | 8.0 | 8.0 | 8.0 | 8.0 |
| Flavor | 0.08 | 0.08 | 0.08 | 0.08 |
| Emulsifier | 0.08 | 0.08 | 0.08 | 0.08 |
| Sodium fluoride | 0.05 | 0.05 | 0.05 | 0.05 |
| Glycerin | 10.0 | 10.0 | 10.0 | 10.0 |
| Sweetener | 0.02 | 0.02 | 0.02 | 0.02 |
| Benzoic acid | 0.05 | 0.05 | 0.05 | 0.05 |
| NaOH | 0.20 | 0.20 | 0.20 | 0.20 |
| Dye | 0.04 | 0.04 | 0.04 | 0.04 |
| Water | Balance to 100% | | | |

In the immediately above formulations, a reduced immunogenic amylase (*e.g*., a variant amylase) is substituted therein with substantially similar results. Also in the Immediately above formulations, any combination of the amylases (*e.g*., variant amylases) can be substituted in with substantially similar results.

| **Lozenge Compositions** | | | | |
|---|---|---|---|---|
| **Component** | **Example No.** | | | |
| | **54** | **55** | **56** | **57** |
| Amylase # | 0.01 | 0.03 | 0.10 | 0.02 |
| Protease # | 0.01 | 0.03 | 0.10 | 0.02 |
| Sorbitol | 17.50 | 17.50 | 17.50 | 17.50 |
| Mannitol | 17.50 | 17.50 | 17.50 | 17.50 |
| Starch | 13.60 | 13.60 | 13.60 | 13.60 |
| Sweetener | 1.20 | 1.20 | 1.20 | 1.20 |
| Flavor | 11.7 | 11.7 | 11.7 | 11.7 |
| Color | 0.10 | 0.10 | 0.10 | 0.10 |
| Corn syrup | Balance to 100% | | | |

In the immediately above formulations a reduced immunogenic amylase (*e.g*., a variant amylase) is substituted therein with substantially similar results. Also in the immediately above formulations, any combination of the amylases useful in the present invention (*e.g*., variant amylases) can be substituted in with substantially similar results.

### 2. Denture Cleaning Compositions

Also provided are In yet additional embodiments, the present invention provides various denture cleaning compositions for cleaning dentures outside of the oral cavity comprise one or more amylase enzymes (*e.g*., variant amylases). Such denture cleaning compositions comprise an effective amount of one or more amylase enzymes (*e.g*., variant amylases), preferably from about 0.0001 % to about 50% of one or more amylase enzymes, more preferably from about 0.001 % to about 35%, more preferably still from about 0.01 % to about 20%, by weight of the composition, and a denture cleansing carrier. Various denture cleansing composition formats such as effervescent tablets and the like are well known in the art (*See e.g.,* US Patent No. 5,055,305) and are generally appropriate for incorporation of one or more amylase enzymes for removing starch-based stains from dentures.

Denture cleaning compositions are illustrated by the following examples.

| **Two-Layer Effervescent Denture Cleansing Table Composition** | | | | |
|---|---|---|---|---|
| **Component** | **Example No.** | | | |
| | **62** | **63** | **64** | **65** |
| Acidic Layer: | | | | |
| Amylase # | 1.0 | 1.5 | 0.01 | 0.05 |
| Tartaric acid | 24.0 | 24.0 | 24.0 | 24.0 |
| Sodium carbonate | 4.0 | 4.0 | 4.0 | 4.0 |
| Sulphamic acid | 10.0 | 10.0 | 10.0 | 10.0 |
| PEG 20,000 | 4.0 | 4.0 | 4.0 | 4.0 |
| Sodium bicarbonate | 24.5 | 24.5 | 24.5 | 24.5 |
| Potassium persulfate | 15.0 | 15.0 | 15.0 | 15.0 |
| Sodium acid pyrophosphate | 7.0 | 7.0 | 7.0 | 7.0 |
| Pyrogenic silica | 2.0 | 2.0 | 2.0 | 2.0 |
| Tetracetylethylene diamine | 7.0 | 7.0 | 7.0 | 7.0 |
| Ricinoleylsulfosuccinate | 0.5 | 0.5 | 0.5 | 0.5 |
| Flavor | 1.0 | 1.0 | 1.0 | 1.0 |
| | | | | |
| Alkaline Layer: | | | | |
| Sodium perborate monohydrate | 32.0 | 32.0 | 32.0 | 32.0 |
| Sodium bicarbonate | 19.0 | 19.0 | 19.0 | 19.0 |
| EDTA | 3.0 | 3.0 | 3.0 | 3.0 |
| Sodium tripolyphosphate | 12.0 | 12.0 | 12.0 | 12.0 |
| PEG 20,000 | 2.0 | 2.0 | 2.0 | 2.0 |
| Potassium persulfate | 26.0 | 26.0 | 26.0 | 26.0 |
| Sodium carbonate | 2.0 | 2.0 | 2.0 | 2.0 |
| Pyrogenic silica | 2.0 | 2.0 | 2.0 | 2.0 |
| Dye/flavor | 2.0 | 2.0 | 2.0 | 2.0 |

In the immediately above formulations a reduced immunogenic amylase (*e.g*., a variant amylase) is substituted therein with substantially similar results. Also in the immediately above formulations, any combination of the amylases (*e.g*., variant amylases) can be substituted in with substantially similar results.

### 3. Personal Cleansing Compositions

Personal cleaning compositions for cleaning the skin may comprise one or more of the amylase enzymes (*e.g*., variant amylases). Such compositions typically comprise from about 0.001% to about 5% amylase enzyme (*e.g*., variant amylases), preferably from about 0.005% to about 2%, and most preferably from about 0.01 % to about 0.8% by weight of the composition. Preferred personal cleansing compositions into which can be included amylase enzymes as described herein include, but are not limited to those described in US Patent Application Ser. Nos. 08/121,623 and 08/121,624. Although various compositions are contemplated, one liquid personal cleaning composition containing a soap component includes (weight %): soap (K or Na) (15.00), 30% laurate, 30% myristate, 25% palmitate, 15% stearate, fatty acids (above ratios) (4.50), Na lauryl sarcosinate (6.00), sodium laureth-3 sulfate (0.66), cocamidopropylbetaine (1.33), glycerine (15.00), propylene glycol (9.00), polyquaternium 10 (0.80), ethylene glycol distearate (EDTA) (1.50), propylparaben (0.10), methylparaben (0.20), amylase # (0.10), protease # (0.10), KOH or NaOH (if necessary to adjust pH), calcium sulfate (3), acetic acid (3), and water (balance to 100).

Personal cleansing bars are also provided. Although various compositions are contemplated, one bar personal cleaning composition containing a soap component includes (weight sodium cocoyl isethionate (47.20), sodium cetearyl sulfate (9.14), paraffin (9.05), sodium soap (*in situ*) (3.67), sodium isethionate (5.51), sodium chloride (0.45), titanium dioxide (0.4), trisodium EDTA (0.1), trisodium etidronate (0.1), perfume (1.20), Na₂ SO₄ (0.87), amylase # (0.10), protease # (0.10), and a mixture of water and minors (balance to 100).

In the immediately above formulations a reduced immunogenic amylase (*e.g*., a variant amylase) is substituted therein with substantially similar results. Also in the immediately above formulations, any combination of the amylases (*e.g*., variant amylases) can be substituted in with substantially similar results.

### EXAMPLE 7

### Wash Performance Test

The wash performance of the variants compositions may be evaluated by any suitable means known in the art. One suitable method for measuring the removal of stain from EMPA 116(blood/milk/carbon black on cotton) cloth swatches (Testfabrics, Inc., Middlesex, N.J. 07030) is described in this Example.

Six EMPA 116 swatches, cut to 3.times.41/2 inches with pinked edges, are placed in each pot of a Model 7243S Terg-O-Tometer (United States Testing Co., Inc., Hoboken, N.J.) containing 1000 ml of water, 15 gpg hardness (Ca++:Mg++::3:1::w:w), 7 g of detergent, and enzyme as appropriate. The detergent base is WFK1 detergent from wfk-Testgewebe GmbH, (Krefeld, Germany) and has the following components (% of final formulation): Zeolite A (25%), sodium sulfate (25%), soda ash (10%), linear alkylbenzenesulfonate (8.8%), alcohol ethoxylate (7-8 EO) (4.5%), sodium soap (3%), and sodium silicate (SiO₂ :Na₂ 0::3.3:1)(3%).

To this base detergent, the following additions are made (% of final formulation): sodium perborate monohydrate (13%), copolymer (Sokalan CP5) (4%), TAED (Mykon ATC Green) (3%), enzyme (0.5%), and whitener (Tinopal AMS-GX) (0.2%).

Sodium perborate monohydrate can be obtained from various commercial sources, including Degussa Corporation, Ridgefield-Park. Sokalan CP5 is obtained from BASF Corporation, Parsippany, N.J. Mykon ATC Green (TAED, tetraacetylethylenediamine) can be obtained from Warwick International, Limited, England. T inopal AMS GX can be obtained from Ciba-Geigy Corporation, Greensboro, N.C.

In one suitable testing method, six EMPA 116 swatches are washed in detergent with enzyme for 30 min at 60° C, rinsed twice for 5 minutes each time in 1000 ml water. Enzymes are added at final concentrations of 0.05 to 1 ppm for standard curves, and 0.25 ppm for routine analyses. Swatches are dried and pressed, and the reflectance from the swatches is measured using the L value on the L*a*b* scale of a Minolta Chroma Meter, Model CR-200 (Minolta Corporation, Ramsey, NJ.). The performance of the test enzyme may be reported as a percentage of the performance of a standard, well-characterized amylase composition.

### EXAMPLE 8

### Variant Amylase Stability in a Liquid Detergent Formulation

This example provides a means for comparison of amylase (*e.g*., variant amylase) stability toward inactivation in a liquid detergent formulation with *Bacillus licheniformis* amylase.

In this method, the detergent formulation for the study is a commercially available laundry detergent (*e.g*., Tide® Ultra liquid laundry detergent (Proctor & Gamble)). Heat treatment of the detergent formulation is necessary to inactivate *in-situ* amylase. This is accomplished by incubating the detergent at 96°C. for a period of 4.5 hours. Concentrated preparations of the *B. licheniformis* amylase and variant (*e.g*., variant amylases) to be tested, in the range of 20 grams/liter enzyme, are then added to the heat-treated detergent, at room-temperature to a final concentration of 0.3 grams/liter enzyme in the detergent formulation. The heat-treated detergent with amylase added is then incubated in a water bath at 50°C. Aliquots are removed from the incubation tubes at 0, 24, 46, 76, and 112 hour time intervals and assayed for enzyme activity by addition to a 1 cm cuvette, containing 1.2 mM of the synthetic peptide substrate suc-Ala-Ala-Pro-phe-p-nitroanilide dissolved in 0.1 M Tris-HCL buffer, pH 8.6, and at 25°C. The initial linear reaction velocity is followed spectrophotometrically by monitoring the absorbance of the reaction product p-nitroanitine at 410 nm as a function of time. The preferred variant(s) are observed to have significantly greater stability towards inactivation than the native *B*. *amyloliquefaciens* enzyme. Estimated half-lives for inactivation in the laundry detergent formulation for the two enzymes are determined under the specified test conditions.

## Claims

1. A method of reducing immunogenicity of a precursor microbial amylase, comprising modifying at least one amino acid at a position which is equivalent to a position in an epitope defined by SEQ ID NO: 5 of *Bacillus licheniformis* amylase having the amino acid sequence of SEQ ID NO: 1 to produce a variant amylase, whereby said variant amylase is less immunogenic than the precursor amylase.

2. The method of claim 1, wherein said precursor microbial amylase is from a member of the genus *Bacillus.*

3. The method of claim 2, wherein said precursor microbial amylase is from *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus* or *B*. *thuringiensis.*

4. The method of claim 3, wherein said precursor microbial amylase has the sequence of SEQ ID NO: 1.

5. The method of any one of claims 1 to 4 wherein said epitope of said precursor amylase is modified by substituting the amino acid sequence of said T-cell epitope with an analogous sequence from a homolog of said amylase, wherein said substitution substantially mimics the major tertiary structure attributes of the T-cell epitope

6. The method of any one of claims 1 to 5 comprising the step of modifying a precursor DNA sequence encoding said precursor amylase to produce a DNA sequence encoding the variant amylase.

## Patentansprüche

1. Verfahren zur Reduzierung der Immunogenität einer mikrobiellen Vorläuferamylase, umfassend Modifizieren zumindest einer Aminosäure an einer Position, die äquivalent ist zu einer Position in einem Epitop, definiert durch SEQ ID NO: 5 von *Bacillus*-*licheniformis*-Amylase mit der Aminosäuresequenz von SEQ ID NO: 1, zwecks Herstellung einer Variantenamylase, wodurch die Variantenamylase weniger immunogen als die Vorläuferamylase ist.

2. Verfahren nach Anspruch 1, wobei die mikrobielle Vorläuferamylase von einem Mitglied der Gattung *Bacillus* ist.

3. Verfahren nach Anspruch 2, wobei die mikrobielle Vorläuferamylase von *B. subtilis*, *B. licheniformis, B. lentus, B. brevis, B. stearothermophilus*, *B. alkalophilus*, *B. amyloliquefaciens, B. clausii*, *B. halodurans*, *B. megaterium*, *B. coagulans*, *B. circulans*, *B. lautus* oder *B. thuringiensis* ist.

4. Verfahren nach Anspruch 3, wobei die mikrobielle Vorläuferamylase die Sequenz von SEQ ID NO: 1 aufweist.

5. Verfahren nach einem der Ansprüche 1 bis 4, wobei das Epitop der Vorläuferamylase modifiziert wird durch Substituieren der Aminosäuresequenz des T-Zell-Epitops mit einer analogen Sequenz aus einem Homolog der Amylase, wobei die Substitution die Tertiärstruktur-Hauptattribute des T-Zell-Epitops im Wesentlichen nachahmt.

6. Verfahren nach einem der Ansprüche 1 bis 5, umfassend den Schritt des Modifizierens einer für die Vorläuferamylase codierenden Vorläufer-DNA-Sequenz, um eine für die Variantenamylase codierende DNA-Sequenz herzustellen.

## Revendications

1. Procédé de réduction de l'immunogénicité d'une amylase précurseur d'origine microbienne, comprenant la modification d'au moins un acide aminé en une position équivalente à une position dans un épitope défini par le SEQ ID NO : 5 de l'amylase de *Bacillus licheniformis* présentant la séquence d'acides aminés de SEQ ID NO : 1 pour produire un variant d'amylase, moyennant quoi ledit variant d'amylase est moins immunogène que l'amylase précurseur.

2. Procédé selon la revendication 1, dans lequel ladite amylase précurseur d'origine microbienne est issue d'un membre du genre *Bacillus*.

3. Procédé selon la revendication 2, dans lequel ladite amylase précurseur d'origine microbienne est issue *de B subtils, B. licheniformis, B. lentus, B. brevis, B. stearothermophilus, B. alkalophilus*, *B amyloliquefaciens*, *B. clausii*, *B. halodurans*, *B. megaterium, B. coagulans*, *B. circulans, B. lautus* ou *B. thuringiensis*.

4. Procédé selon la revendication 3, dans lequel ladite amylase précurseur d'origine microbienne présente la séquence de SEQ ID NO : 1.

5. Procédé selon l'une quelconque des revendications 1 à 4, dans lequel ledit épitope de ladite amylase précurseur est modifié par substitution de la séquence d'acides aminés dudit épitope de lymphocyte T par une séquence analogue d'un homologue de ladite amylase, ladite substitution imitant sensiblement les attributs majeurs de structure tertiaire de l'épitope de lymphocyte T.

6. Procédé selon l'une quelconque des revendications 1 à 5, comprenant l'étape de modification d'une séquence d'ADN précurseur codant pour ladite amylase précurseur pour produire une séquence d'ADN codant pour le variant d'amylase.
